(19) Europäisches Patentamt
European Patent Office
Office européen des brevets

(11) **EP 2 839 828 A1**

(12) **EUROPEAN PATENT APPLICATION**

(43) Date of publication:
**25.02.2015 Bulletin 2015/09**

(21) Application number: **13181571.4**

(22) Date of filing: **23.08.2013**

(51) Int Cl.:
*A61K 9/00* (2006.01) *A61K 9/06* (2006.01)
*A61K 31/34* (2006.01) *A61P 17/02* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **NITTO DENKO CORPORATION**
**Ibaraki-shi, Osaka 567-8680 (JP)**

(72) Inventors:
- **Amiguet Vercher, Sandra**
  **1010 Lausanne (CH)**
- **De Visscher, Geofrey**
  **1071 Chexbres (CH)**
- **Eevers, Walter**
  **3520 Zonhoven (BE)**
- **Schüwer, Nicolas**
  **1007 Lausanne (CH)**
- **Shigematsu, Takayuki**
  **1814 La Tour-de-Peilz (CH)**
- **Takeiri, Mayu**
  **1025 St. Sulpice (CH)**
- **Hubbell, Jeffrey A.**
  **1028 Préverenges (CH)**
- **Tortelli, Federico**
  **1004 Lausanne (CH)**

(74) Representative: **Hoffmann Eitle**
**Patent- und Rechtsanwälte PartmbB**
**Arabellastraße 30**
**81925 München (DE)**

(54) **Compunds and formulations for the treatment of wounds**

(57) The present invention relates to the treatment of wounds. More particularly, it relates to compounds according to Formula 1 for use in methods of promoting wound healing, to formulations which incorporate these compounds and to a method of treating wounds using such compounds and formulations:

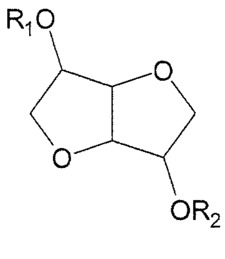

Formula (1)

wherein $R_1$ and $R_2$ may be the same or different and are selected from the group consisting of hydrogen (H) and a physiologically hydrolyzable chemical group consisting of alkylcarbonyl, alkenylcarbonyl arylcarbonyl, heteroarylcarbonyl, alkoxycarbonyl, aryloxycarbonyl, heteroaryloxycarbonyl, alkylether, alkenylether, arylether, and heteroarylether groups wherein the alkyl moiety consists of unsubstituted or substituted, straight-chain or branched-chain and cyclic alkyl groups having 2-22 carbon atoms, wherein the alkenyl moiety consists of unsubstituted and substituted, straight-chain or branched-chain and cyclic alkenyl groups having 2-22 carbon atoms, wherein the aryl moiety consists of unsubstituted and substituted phenyl, and phenalkyl groups wherein the alkyl moiety contains 1-3 carbon atoms and the phenyl moiety is unsubstituted or substituted, and the heteroaryl moiety is an aromatic 5- or 6-membered heterocyclic ring containing one or two heteroatoms selected from the group consisting of nitrogen, oxygen, and sulphur.

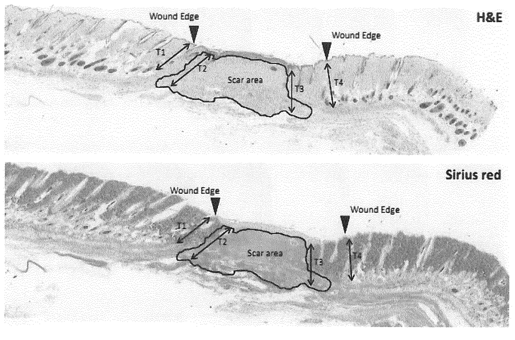


FIG. 4

EP 2 839 828 A1

Printed by Jouve, 75001 PARIS (FR)

## Description

### 1. Field of the Invention

The present invention relates to the treatment of wounds. More particularly, it relates to compounds promoting wound healing, to formulations which incorporate these compounds and to a method of treating wounds using such compounds and formulations.

### 2. Background of the Invention

#### *2.1 Pathophysiology of scar tissue formation*

The healing of wounds (e.g. dermal wounds) is a complex process involving the collaboration of several tissues and cell lineages. This healing is mediated by local cells such as dermal fibroblasts and keratinocytes. However, cells from the immune system also play a crucial role in regulating the healing by releasing soluble factors influencing nearby cells but also by modulating the extracellular matrix (ECM). Consequently re-epithelialization is crucial as is the regeneration of an 'adequate' ECM. In terms of scarring this repaired ECM should not be bulky, provide adequate strength and also be organised comparable to the normal skin tissue. In this last part collagens play an important role.

The primary objective of the healing process is to fill the gap created by tissue destruction and restore the structural continuity of the injured part. The gap is normally filled with a fibrous tissue otherwise known as scar tissue. With the exception of very minor and superficial lesions, every wound (e.g. after accident, disease, or surgery) results in some degree of scarring. The biological repair process differs little from one kind of tissue to another and is generally the same for all acute wounds, open or closed, regardless of how they are caused. The normal wound healing process is a cascade of overlapping events that are commonly divided into four phases: the coagulation phase, the inflammatory phase, the proliferative phase and the remodelling phase.

The initial coagulation phase provides haemostasis by depositing a fibrin clot which also comprises platelets. This clot will provide a first extracellular matrix aiding the cells of the later phases to move in and repair the damage. When tissue is first wounded, a vascular spasm causes blood vessels in the vicinity of the wound to constrict thereby minimising blood loss. Exposure of blood to proteins in the tissue (e.g. thromboplastin) initiates changes to blood platelets and the soluble plasma protein fibrinogen. Platelets immediately form a plug at the site of injury (primary haemostasis) while soluble fibrinogen is converted via a complex cascade to insoluble fibrin strands which strengthen the platelet plug (secondary haemostasis). Fibrin and fibronectin cross-link together to form a plug that provides the main structural support for the wound until collagen is deposited. The platelets will provide an initial surge of factor that will initiate and steer the inflammatory reaction.

The major function of the inflammatory phase is to remove damaged tissue and foreign matter from the wound and fight infectious agents, thus preparing the wound environment for healing. Vasoconstriction lasts five to ten minutes and is followed by vasodilation, a widening of blood vessels, which peaks at about 20 minutes post-wounding. Vasodilation is accompanied by small vessel permeability in the region of the wound. The resulting oedema in the area of the injury is the source of the familiar pain and swelling which occurs early after injury. The increased porosity of blood vessels facilitates the entry of circulating white blood cells such as polymorphonuclear neutrophils (PMN) and monocytes from the bloodstream into the wound site. PMN initially appear in the wound shortly after injury and their numbers increase steadily, peaking at about 24 to 48 h. They function as a first responder and engulf and degrade any bacteria in the wound in a process called phagocytosis. Monocytes/macrophages are also attracted to the wound site by growth factors released by platelets and other cells and, once in the wound site, mature into macrophages where they supersede PMN as the major wound phagocyte. These macrophages also release growth factors such as platelet-derived growth factor (PDGF) and transforming growth factor beta (TGF-β). These growth factors attract and activate fibroblasts from surrounding tissue into the wound where they then proliferate.

The arrival of fibroblasts (about 2 to 5 days post wounding) marks the beginning of the proliferative phase of wound healing where the focus moves to the building of new tissue to fill the wound space. The proliferative phase is characterized by the formation of granulation tissue in the wound that serves as the foundation for scar tissue development. Granulation tissue functions as rudimentary tissue, and begins to appear in the wound already during the inflammatory phase and continues growing until the wound bed is covered. Granulation tissue consists of a combination of cellular elements, including fibroblasts and inflammatory cells, along with new capillaries embedded in a loose extracellular matrix of collagen, fibronectin and hyaluronan. Fibroblasts are the primary synthetic element in the repair process and are responsible for the production of the majority of structural proteins used during tissue reconstruction. In particular, fibroblasts produce large quantities of collagen, a family of triple-chain glycoproteins, which form the main constituent of the extracellular wound matrix and which are ultimately responsible for imparting tensile strength to the scar. Even as fibroblasts are producing new collagen, collagenases and other factors degrade it. Shortly after wounding, the rate

of synthesis of collagen exceeds its degradation so collagen levels in the wound rise, but later production and degradation become equal so there is no net collagen gain. The fibroblasts also secrete growth factors that attract epithelial cells to the wound site. Angiogenesis, or neovascularization, occurs concurrently with fibroblast proliferation and the formation of capillaries in the granulation tissue allows vital oxygen and nutrients to be delivered to the wound site. The final component of the proliferative phase is epithelialization which is the regeneration, migration, proliferation and differentiation of epithelial cells at the wound's edge to form a new surface area similar to that destroyed by the injury. By the end of the proliferative stage white blood cells leave the wound site, oedema diminishes, and the wound begins to blanch as the small blood vessels become occluded by thrombosis and degenerate.

The fourth phase of wound healing is the remodelling phase which begins about three weeks post wound and can continue for six months or longer. The remodelling phase is generally said to begin when the levels of collagen production and collagen degradation equalize. During this phase, type III collagen, which is prevalent during the proliferative phase, is replaced by type I collagen thus forming the final scar tissue. The collagen fibrils, which previously were randomly oriented, align in the direction of mechanical tension, providing further mechanical strength to the wound. Upon completion of the entire process, the skin regains its chemical and physical barrier functions. Clinically, the scar becomes avascular and the scar tissue may achieve up to 70-80% of tensile strength of normal tissue by the end of three months. A review of the processes involved in wound healing was published by Singer and Clark (1999).

*2.2 Problems with scar tissue*

Scar tissue formation is a natural part of the healing process after wound injury, and while it is often desirable to increase the rate of healing for acute and chronic wounds, in some instances the regulation of scar formation is of primary importance and the rate of wound healing is a secondary consideration. In some cases, an exaggerated healing response can result in the production of copious amounts of scar tissue (e.g. hypertrophic and keloid scarring). Excessive scarring can be a major medical problem, often resulting in loss of function, restriction of tissue movement and/or growth. For instance, sweat glands or hair follicles do not exist in scar tissue. This can impair the regulation of body temperature, particularly around the scar area. Moreover, scars can reduce the flexibility of the tissue in which they form. For instance, scarring can reduce movement of the skin on the neck, shoulders and joints such as the jaws, elbows and knee caps. Scarring can also have adverse aesthetic effects (e.g. discoloration) which in some cases may lead to adverse psychological effects. Social, emotional and psychological effects of scars are especially notable on individuals with visible scars.

*2.3 Prior art treatments*

Topical agents comprising sugar have been used to treat wounds for a long time. In general, honey (whose main constituents are glucose, fructose and maltose), sugar (sucrose), and molasses (whose main constituents are sucrose and glucose) have been used (Jorge Chirife et al. 1983).

In the 1980's two articles reviewed these practices and showed that honey was frequently used by ancient civilizations such as the Egyptians (Forrest 1982a, 1982b). The wound healing properties of honey has been attributed to its high sugar concentration (about 84% fructose and glucose). This high sugar content draws fluid from the surrounding tissue by osmosis. The osmotic action can also partly explain the bactericidal activity of honey as can the low pH of honey (about 3.2 to 4.5) which has also been cited as a reason for its wound healing properties. However, the microbiological effectiveness of honey varies widely depending on its origin. For instance, honey prepared from the nectar of certain plants such as conifer or manuka (Leptospermum scoparium) shows particularly high antibacterial activity whereas for some other honeys this effect is not detectable. Other disadvantages of natural honey are its lack of standardization and the possible contamination with pesticides, antibiotics or clostridial spores. Several medicinal honey-containing wound dressings are commercially available: HoneySoft™ (Taureon, The Netherlands), InfectoHoney™ (Infectopharm, Germany), Medihoney™ (Comvita, New Zealand), Medihoney Gel™ dressing, Medihoney™ gauze and MelMax™ (Dermagenics, The Netherlands).

In 1980 Herszage et al. published a report of a non-controlled, non-randomized 120 patient study, where infected and other superficial wounds treated with sugar showed a 99.2% curing rate (Jorge Chirife et al. 1983). 605 patients were treated with granulated sugar and povidone-iodine for wounds, bums and ulcers (Knutson et al. 1981). Treating a wide variety of difficult wounds this study concluded that the sugar and povidone iodine combination outperformed other products. In another study 19 patients with post-operative mediastinitis received treatment of the infected wound with granulated sugar (Trouillet et al. 1985). In this report, packing the mediastinal cavity with granulated sugar every 3 to 4 hours resulted in wound debridement, rapid granulation tissue deposit and wound sterilization after 7 days. Applying granulated sugar to wounds results in sugar concentrations close to saturation (6.57 M) (J Chirife, Scarmato, and Herszage 1982; Jorge Chirife et al. 1983). The proposed mode of action is not entirely elucidated.

More recently Archer and coworkers (1990) published a comparative study in pigs with full thickness incisional wounds. In one treatment group the wounds were packed with a custom made paste (composition: 1200 g castor sugar,

1800 g icing sugar, 686 ml PEG 400 and 19 ml 30% $H_2O_2$, pH 4.8). The other wounds were treated with 0.2% Chlorhexidine gluconate, 0.2% Triclosan, 0.8% povidone iodine or EUSOL half strength (sodium hypochloride). In their conclusion the sugar paste was preferred over the other treatments. In another rat wound model where sucrose was delivered by means of an implanted sponge no beneficial effect was seen (Kössi et al. 2000). However there are some inconsistencies with this study as neither sucrose nor its metabolites glucose and fructose were significantly increased in the wound fluid. Even the glucose levels decreased in the sucrose treated groups. These effects have not been explained and may have severely influenced that study's outcome.

For example, an acidic pH at the wound surface caused by the sugar solution could be contributing to the healing process (Archer et al. 1990). At least two studies have linked low pH (up to 3.5) to increased epithelialization (Kaufman et al. 1985) and wound healing in general (Leveen et al. 1973). The sugar paste used by Archer and coworkers (1990) had a pH of 4.8 at 10 to 50% concentrations in water. Another contributing factor is low water activity, also meaning high osmotic pressure, caused by high sugar concentrations (Jorge Chirife et al. 1983). Through this mechanism it acts as an antibacterial solution, not through a direct action, but merely by causing osmotic stress. For example, it was shown that *complete in vitro* growth of *S. aureus* could be achieved with a 183g/100g water solution. This is below what one would expect in the treatment with granulated sugar (225g/100g water @ 37°C). Other bacteria showed to be less resistant to sugar and therefore require lower sugar concentrations. An important distinction to be made is the fact that the first mechanism is potentially universal in wound healing, whereas the latter only applies to infected wounds.

A wound healing function has also been ascribed to other saccharides, such as e.g. D-ribose or oligo- or polysaccharides derived from glucomannan (Patent document 1: US Patent Application Publication No. US 2004/127428 A1 and Patent document 2: International PCT Application Publication No. WO 2009/043111). In these applications the node of action is clearly different as compared to the application of bulk sugars to the wound bed. D-ribose probably acts as a precursor in the ATP generating pathway which normally has glucose as a substrate and D-ribose as an intermediate step. Glucomannan is a konjac root (Amorphophallus konjac) derived polysaccharide (200-2000 kDa) comprising glucose and mannose residues as a basic GGMMGMMMMMGGM repeat, which is approved as a food additive. Despite these, there remains a constant need for further effective agents for the promotion of wound healing and scarring improvement.

Phase 3 efficacy trials in skin graft donor sites have allegedly showed that wounds treated with a topical formulation of the sugar Mannose-6-Phosphate (Juvidex®) had an appearance that more closely resembled normal skin compared to placebo.

Certain vitamin and mineral deficiencies have also been associated with decreased wound healing (e.g., deficiencies of vitamins A, C and D; and calcium, magnesium, copper, and iron) but there is no strong evidence that increasing the serum levels of these substances above their normal levels actually enhances wound healing. Vitamin E cream is sometimes recommended for the self-management of scars although again there is no medical evidence to suggest that it has any effect.

While topical agents comprising sugar have been used for wound healing, none have been shown to reduce scarring or to promote the regeneration of normal tissue and/or promote the generation of normal tissue/skin structures. Where scars have already formed, existing therapeutic strategies for their treatment include pressure therapy, silicone gel sheeting, intra-lesional TAC, cryosurgery, radiation, laser therapy, INF, 5-FU and surgical excision. However, these therapeutic strategies have the disadvantage of only treating scars that have already formed. It would be better to reduce the amount of scarring that occurs in the first place.

## 3. Summary of the Invention

The present invention relates to the treatment of wounds, to compounds for use in methods of treating wounds, to formulations which incorporate these compounds and to methods of treating wounds using these compounds and formulations. In particular, the invention provides for reduced scarring during the wound healing process, to regeneration of normal tissue after wounding and to the generation of normal tissue and/or skin structures after wounding.

The present invention aims to overcome the deficiencies of the state of the art wound treatments discussed above. The problem is solved by administering a compound according to Formula (1) to the wound of a patient in need thereof:

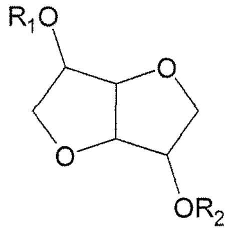

Formula (1)

wherein $R_1$ and $R_2$ may be the same or different and are selected from the group consisting of hydrogen (H) and a physiologically hydrolyzable chemical group consisting of alkylcarbonyl, alkenylcarbonyl arylcarbonyl, heteroarylcarbonyl, alkoxycarbonyl, aryloxycarbonyl, heteroaryloxycarbonyl, alkylether, alkenylether, arylether, and heteroarylether groups wherein the alkyl moiety consists of unsubstituted or substituted, straight-chain or branched-chain and cyclic alkyl groups having 2-22 carbon atoms, wherein the alkenyl moiety consists of unsubstituted and substituted, straight-chain or branched-chain and cyclic alkenyl groups having 2-22 carbon atoms, wherein the aryl moiety consists of unsubstituted and substituted phenyl, and phenalkyl groups wherein the alkyl moiety contains 1-3 carbon atoms and the phenyl moiety is unsubstituted or substituted, and the heteroaryl moiety is an aromatic 5- or 6-membered heterocyclic ring containing one or two heteroatoms selected from the group consisting of nitrogen, oxygen, and sulphur. Cyclic alkyl groups and cyclic alkenyl groups also encompass groups having a cyclic ether, such as an epoxide, in their structure.

Preferred compounds are those where $R_1$ and $R_2$ are the same or different and selected from the group consisting of H or $-COR_3$, wherein $R_3$ is a C2 to C22 hydrocarbon group. More preferred are those compounds where $R_1$ and $R_2$ are the same and selected from the group consisting of H or $-COR_3$, wherein $R_3$ is a linear C6, C7, C8, C9 or C10 alkyl group. In each case the compound may be any of the possible stereoisomers (e.g. enantiomers or diasteriosomers) of Formula (1). The compound according to Formula (1) can be formulated as a topical formulation and the topical formulation may comprise more than one compound according to Formula (1).

Topical formulations may comprise more than one compound according to Formula (1). For example, the formulation may comprise isosorbide and a monoester according to Formula (1). The formulation may comprise isosorbide and a diester according to formula (1) where $R_1$ and $R_2$ of the diester are the same or different. The formulation may comprise two different monoesters according to Formula (1). The formulation may comprise two different diesters according to Formula (1) where $R_1$ and $R_2$ are the same or different for either of the diesters. The formulation may comprise a monoester and a diester where $R_1$ and $R_2$ of the diester are the same or different. Isosorbide can also be included in the mono-/mono-, di-/di-, or mono-/diester formulations mentioned above. For each of the mixed isosorbide/monoester, isosorbide/diester, mono-/mono-, di-/di-, or mono-/di- ester formulations mentioned above, it is preferred that $R_3$ (of $R_1$ and $R_2$) is a linear C6, C7, C8, C9 or C10 alkyl group. For the present invention the only active agent(s) in the formulation may be a single compound or mix of compounds according to Formula (1). If necessary, additional active agents may be included in the formulation as explained below.

The present invention also relates to a composition comprising isosorbide and at least one compound according to Formula (1), preferably Formula (1a):

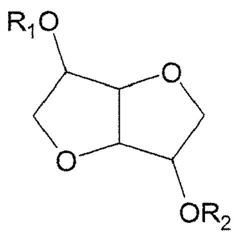

Formula (1)

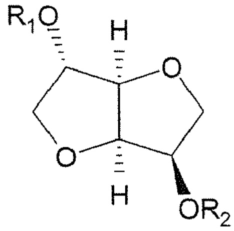

Formula (1a)

wherein $R_1$ and $R_2$ are the same or different and are selected from the group consisting of hydrogen (H) and a physiologically hydrolyzable chemical group consisting of alkylcarbonyl, alkenylcarbonyl arylcarbonyl, heteroarylcarbonyl, alkoxycarbonyl, aryloxycarbonyl and heteroaryloxycarbonyl, alkylether, alkenylether, arylether, and heteroarylether groups wherein the alkyl moiety consists of unsubstituted or substituted, straight-chain or branched-chain and cyclic alkyl groups having 2-22 carbon atoms, wherein the alkenyl moiety consists of unsubstituted and substituted, straight-chain or branched-chain and cyclic alkenyl groups having 2-22 carbon atoms, wherein the aryl moiety consists of unsubstituted and substituted phenyl, and phenalkyl groups wherein the alkyl moiety contains 1-3 carbon atoms and the phenyl moiety is unsubstituted or substituted, and the heteroaryl moiety is an aromatic 5- or 6-membered heterocyclic ring containing one or two heteroatoms selected from the group consisting of nitrogen, oxygen, and sulphur, provided that at least one of $R_1$ and $R_2$ is a physiologically hydrolyzable chemical group. Cyclic alkyl groups and cyclic alkenyl groups also encompass groups having a cyclic ether, such as an epoxide, in their structure.

Surprisingly, it has been found that when compounds according to Formula (1) or topical formulations containing these compounds are applied to a wound the amount of scar tissue formed after wound healing is significantly reduced. Even more surprisingly, the scar tissue that does form exhibits characteristics more closely associated with normal functioning tissue. In a mouse model normal structures such as hair follicles and sebaceous glands can be observed at the scar position. Thus, not only do the compounds of the invention reduce scarring, but they also promote the regeneration of normal tissue and promote the generation of normal tissue and/or skin structures.

Thus, the present invention further relates to methods of treating wounds, methods of reducing scar formation, methods for regenerating normal tissue and methods for promoting the generation of normal tissue and/or skin structures. The present invention also relates to cosmetic methods of treating wounds and cosmetic methods of reducing scar formation.

The wound to be treated can be an acute wound or a chronic wound. The wound may be an open wound or a closed wound. The compounds and the formulations of the invention are particularly useful for treating wounds created during surgery.

When the compounds and the formulations of the invention are used in methods for treating wounds a reduction in scar formation can be observed. Thus, the compounds and the formulations of the invention can be used to reduce the formation of scars such as normal scars, hypertrophic scars, keloid scars, atrophic scars or striae (stretch marks).

Other objects, effects and advantages of the present invention will be apparent from the following description.

## 4. Brief Description of the Figures

**Figure 1** is a schematic representation of the procedure in the animal model for scar analysis.

**Figure 2** shows the macroscopic tissue images of 5-day-, 10-day-, and 15-day-postoperative wounds.

**Figure 3** shows the outcome of the macroscopic wound size evaluation on controls and isosorbide and polysorb treated wounds.

**Figure 4** shows a H&E image and the corresponding Sirius red image illustrating the measurements used for scar size analysis.

**Figure 5** shows the results of the quantitative scar index analysis. Isosorbide is significantly different from Control and Vehicle ($p<0.05$).

**Figure 6** shows the Sirius red images of the wounds treated with the composition of the present invention in comparison with polysorb or isosorbide treated wounds and the control.

**Figure 7** shows a screen shot of the analysis tool automatically detecting collagen and organised collagen for quantitative scar index image analysis as discussed below in the Examples.

**Figure 8** shows collagen area and percentage of organization within that collagen as measured by image analysis.

**Figure 9** shows a post-operative overview image of a mouse with four wounds as indicated and covered with op-film.

## 5. Detailed Description of the Preferred Embodiments

### *5.1 Definitions*

A "wound" as used in the present invention is to be understood as a disruption of tissue integrity. For instance, wounds may comprise minor cuts or abrasions; complicated/full thickness wounds; traumatic wounds such as e.g. abrasions, lacerations; surgical wounds; post-surgical incisions; or chronic/non-healing wounds like pressure sores; diabetic ulcers; injuries to connective tissue like bone or cartilage; burn injuries; and scars (e.g. keloid scars, contracture scars, hypertrophic scars and acne scars). The wound may be an open wound or a closed wound. A 'closed' wound in the sense of the present invention means a wound that was open at one point (e.g. a surgical incision or an accidental cut) and that has been purposefully closed by means of a suture, staple, surgical adhesive and the like.

A "scar" in the sense of the present invention means normal scars, hypertrophic scars, keloid scars, contracture scars, atrophic scars and striae. Symptoms of scars include skin discolorations (including redness, changes in pigmentation, or other discolorations e.g. from blanching), erythema, dry, flaky, or itchy skin, raised area above the surrounding skin, keloid formation, hypertrophy, scar pain, decreased vascularization of the scar and/or surrounding tissue, reduced pliability, and poor aesthetic appearance (including quality and texture of the scar tissue). Thus, for the purpose of the present invention, the reduction of scarring can also be considered as the treatment (prevention or amelioration) of these symptoms.

"Topical administration" in the sense of the present invention means administration to a definite surface, e.g. directly to the wound surface, or if the wound has been closed, to the area around the closure.

### *5.2 Active Compounds*

The compounds suitable for practising the invention are those according to Formula (1):

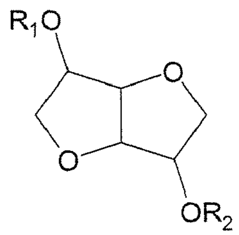

Formula (1)

wherein $R_1$ and $R_2$ may be the same or different and are independently selected from the group consisting of H and a physiologically hydrolyzable chemical group consisting of alkylcarbonyl, alkenylcarbonyl arylcarbonyl, heteroarylcarbonyl, alkoxycarbonyl, aryloxycarbonyl, heteroaryloxycarbonyl, alkylether, alkenylether,

arylether, and heteroarylether groups, wherein the alkyl moiety consists of unsubstituted or substituted, straight-chain or branched-chain and cyclic alkyl groups having 2-22 carbon atoms, wherein the alkenyl moiety consists of unsubstituted and substituted, straight-chain or branched-chain and cyclic alkenyl groups having 2-22 carbon atoms, wherein the aryl moiety consists of unsubstituted and substituted phenyl, and phenalkyl groups wherein the alkyl moiety contains 1-3 carbon atoms and the phenyl moiety is unsubstituted or substituted, and the heteroaryl moiety is an aromatic 5- or 6-membered heterocyclic ring containing one or two heteroatoms selected from the group consisting of nitrogen, oxygen, and sulphur.

Preferred compounds are those where $R_1$ and $R_2$ are the same or different and selected from the group

consisting of H or $-COR_3$, wherein $R_3$ is a C2 to C22 hydrocarbon group. More preferred are those compounds where $R_1$ and $R_2$ are the same and selected from the group consisting of H or $-COR_3$, wherein $R_3$ is a linear C6, C7, C8, C9 or C10 alkyl. In each case the compound may be any of the possible stereoisomers (e.g. enantiomers or diasterisomers) of Formula (1). It is a preferred embodiment of the present invention that the compound of Formula (1) is a compound having the stereochemistry shown in the following Formula (1a):

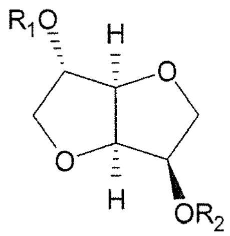

Formula (1a)

wherein also in this case $R_1$ and $R_2$ may be the same or different and are independently selected from the group consisting of hydrogen (H) and a physiologically hydrolyzable chemical group consisting of alkylcarbonyl, alkenylcarbonyl arylcarbonyl, heteroarylcarbonyl, alkoxycarbonyl, aryloxycarbonyl, heteroaryloxycarbonyl, alkylether, alkenylether, arylether, and heteroarylether groups, wherein the alkyl moiety consists of unsubstituted or substituted, straight-chain or branched-chain and cyclic alkyl groups having 2-22 carbon atoms, wherein the alkenyl moiety consists of unsubstituted and substituted, straight-chain or branched-chain and cyclic alkenyl groups having 2-22 carbon atoms, wherein the aryl moiety consists of unsubstituted and substituted phenyl, and phenalkyl groups wherein the alkyl moiety contains 1-3 carbon atoms and the phenyl moiety is unsubstituted or substituted, and the heteroaryl moiety is an aromatic 5- or 6-membered heterocyclic ring containing one or two heteroatoms selected from the group consisting of nitrogen, oxygen, and sulphur.

Also within the scope of this preferred embodiment, the more preferred compounds are those where $R_1$ and $R_2$ are the same or different and selected from the group consisting of H or $-COR_3$, wherein $R_3$ is a C2 to C22 hydrocarbon group. Even more preferred are those compounds where $R_1$ and $R_2$ are the same and selected from the group consisting of H or $-COR_3$, wherein $R_3$ is a linear C6, C7, C8, C9 or C10 alkyl.

***5.2.1 $R_1$ and $R_2$ are both H***

A preferred compound according to Formula (1) is one where $R_1$ and $R_2$ are both hydrogen (H). An example of such a compound is isosorbide which is a preferred compound for the present invention. Isosorbide (CAS [652-67-5], entry No. 5245 in the $13^{th}$ Ed. of The Merck Index) is a white crystalline solid that can be prepared by double dehydration of sorbitol. Isosorbide is readily available on industrial scales, even in pharmaceutical grade, starting from e.g. cellulose or starch, originating from cereals, corn or other sources. Isosorbide may be produced easily and at low costs, especially when compared to oligo- or polysaccharides, previously described in the context of wound healing that are often only accessible by means of enzymatic in vitro-synthesis.

Isosorbide has been used in Japan for the treatment of patients suffering from tinnitus, vertigo and Meniere's disease (Miyagawa et al. 2009; Nozawa et al. 1995). Isosorbide has also been reported as an adjunct for controlling intraocular pressure (US 2004/0001821). Regarding tolerance and toxicity isosorbide has been used in large oral doses of 70-140 ml/day in syrup or gel containing 70% isosorbide (Isobide™ & Ismotic™) and has been administered to humans for several indications where decreased pressure by diuretic action was required.

*5.2.2 Derivatives*

When either one or both of $R_1$ and $R_2$ is not hydrogen (H) then the compound of Formula (1) can be considered to be a derivative for the purposes of this invention. The derivatives to be employed in accordance with the invention are physiologically hydrolysable derivatives, i.e. derivatives wherein one or both of the hydrogen atoms of the hydroxyl groups are replaced with moieties which give rise to a compound which is hydrolysable (i.e. capable of reacting back to the respective hydroxyl-containing compound) under physiological conditions, typically conditions in or near a wound or on top of scar tissue. The physiologically hydrolysable chemical groups of the invention are independently selected from the group consisting of alkylcarbonyl, alkenylcarbonyl, arylcarbonyl, heteroarylcarbonyl, alkoxycarbonyl, aryloxycarbonyl, heteroaryloxycarbonyl, alkylether, alkenylether, arylether, and heteroarylether groups, wherein the alkyl moiety

attached to the carbonyl group or oxygen atom may be unsubstituted or substituted; it may be a C2-22 straight-chain alkyl group or a C3-C22 branched-chain alkyl group or a C3-22 cyclic alkyl group, wherein the alkenyl moiety may be unsubstituted or substituted; it may be a C2-22 straight-chain alkenyl group or a C3-22 branched-chain alkenyl group or a C4-22 cyclic alkenyl group, wherein the aryl moiety is selected from the group that consists of unsubstituted and substituted phenyl, and phenalkyl groups wherein the alkyl moiety contains 1-3 carbon atoms and may optionally be substituted and the phenyl moiety is unsubstituted or substituted, and the heteroaryl moiety is an unsubstituted or substituted aromatic 5- or 6-membered heterocyclic ring containing one or two heteroatoms selected from the group consisting of nitrogen, oxygen, and sulphur.

Suitable examples of such hydrolysable derivatives are thus monoester derivatives, diester derivatives, monoether derivatives and diether derivatives, as well as mono-and di-carbonates and compounds with mixed hydrolysable groups (e.g., a compound of formula (1), carrying one alkylester group and one alkylether group). Monoester derivatives and diester derivatives (where in each instance the individual residues may be same or different) are preferred. Among these preferred ester compounds, those having one or two alkyl groups or alkenyl groups, each with 6, 7, 8, 9 or 10 carbon atoms, are particularly preferred. Preferred examples include derivatives derived from fatty acids, including fully saturated as well as unsaturated fatty acids, amino acids as well as other organic compounds comprising a carboxyl group which may form an ester derivative with the hydroxyl group.

As indicated above, it is a preferred embodiment of the invention to use a compound of Formula (1a). Also for this preferred embodiment physiologically hydrolyzable chemical groups should be used that are independently selected from the group consisting of alkylcarbonyl, alkenylcarbonyl arylcarbonyl, heteroarylcarbonyl, alkoxycarbonyl, aryloxycarbonyl, heteroaryloxycarbonyl, alkylether, alkenylether, arylether, and heteroarylether groups, wherein the alkyl moiety attached to the carbonyl group or oxygen atom may be unsubstituted or substituted; it may be a C2-22 straight-chain alkyl group or a C3-C22 branched-chain alkyl group or a C3-22 cyclic alkyl group, wherein the alkenyl moiety may be unsubstituted or substituted; it may be a C2-22 straight-chain alkenyl group or a C3-22 branched-chain alkenyl group or a C4-22 cyclic alkenyl group, wherein the aryl moiety is selected from the group that consists of unsubstituted and substituted phenyl, and phenalkyl groups wherein the alkyl moiety contains 1-3 carbon atoms and may optionally be substituted and the phenyl moiety is unsubstituted or substituted, and the heteroaryl moiety is an unsubstituted or substituted aromatic 5- or 6-membered heterocyclic ring containing one or two heteroatoms selected from the group consisting of nitrogen, oxygen, and sulphur.

Also for the compounds of Formula (1a), ester compounds are preferred, especially those having one or two alkyl groups or alkenyl groups, each with 6, 7, 8, 9 or 10 carbon atoms. Preferred examples include derivatives derived from fatty acids, including fully saturated as well as unsaturated fatty acids, amino acids as well as other organic compounds comprising a carboxyl group which may form an ester derivative with the hydroxyl group.

When either of $R_1$ and $R_2$ is $-COR_3$ then the compound is an ester derivative. For instance, where the core structure is isosorbide and only one of $R_1$ or $R_2$ is $-COR_3$ then the compound is an isosorbide monoester. If both $R_1$ and $R_2$ are $-COR_3$ then the compound is an isosorbide diester.

For the derivatives according to Formula (1), including those having an isosorbide core structure of Formula (1a), $R_3$ is preferably a linear C2 to C22 hydrocarbon group that may be saturated or unsaturated. $R_3$ may be the same or different for $R_1$ and $R_2$. Preferably, $R_3$ is a linear or branched C6 to C10 alkyl radical. More preferably $R_3$ is the same for both $R_1$ and $R_2$ and is a linear or branched C6 to C10 alkyl radical. Even more preferably, $R_3$ is the same for both $R_1$ and $R_2$ and is a linear C6, C7, C8, C9 or C10 alkyl radical.

Mono- and diesters of isosorbide have been known for some time and are generally used as plasticizers to increase plasticity or fluidity of polymeric materials, inks and varnishes. US 2,322,821 describes the preparation of compositions of isosorbide monoesters (and other stereoisomers). US 2011/196161 describes the preparation of isosorbide diesters. US 2009/0301348 (see Examples 2 to 6) also describes isosorbide diesters that are particularly useful for this invention. Monoesters such as isosorbide laurate and diesters such as isosorbide dicaprylate have been used as emollients and skin-conditioning agents in cosmetic applications. Dimethyl isosorbide is an isosorbide diether that has been used as a skin penetration enhancer (WO 2012/131348; WO 2005/049025; US 2007/179121) and as a volatile coagent (US 2010/0322875). The use of monoalkyl-, dialkyl-, monoalkanoyl- and dialkanoyl-isosorbides for the amelioration, reduction and/or reversal of mammalian skin aging, particularly improving hydration, has been reported in WO 2011/059866 A2. The use of isosorbide 2-ethylhexanoic acid diester in a cleansing cream has also been reported (JP 59-175408).

A typical preparation method is by an esterification reaction of isosorbide with a short-, medium-, or long-chain fatty acid. The fatty acid can have a straight or branched chain and can be saturated or unsaturated. The fatty acid may contain a cyclic ether, such as epoxide, in its structure. Examples of saturated fatty acids include propionic acid, butyric acid, isobutyric acid, valeric acid, isovaleric acid, caproic acid, enanthic acid, caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, heneicosylic acid, behenic acid and tricosylic acid. Examples of unsaturated fatty acids include myristoleic acid, palmitoleic acid, sapienic acid, oleic acid, elaidic acid, vaccenic acid, linoleic acid, linoelaidic

acid, α-linolenic acid, arachidonic acid, eicosapentaenoic acid, erucic acid and vernolic acid. The fatty acid is not particularly limited provided that it can lead to a compound according to Formula (1) and preferably Formula (1a).

Isosorbide diesters are also commercially available. For example, mixtures of isosorbide di(octanoic acid)ester and isosorbide di(decanoic acid)ester can be obtained from Roquette (France) under the trade name Polysorb ID 37®. This diester is particularly preferred for use in the present invention. Dioctanoyl isosorbide is also available from Sytheon Ltd. under the trade name Synovea® DOI. This preparation can also be used for the purposes of the present invention.

Those ester derivatives of isosorbide that are oils (e.g. where $R_3$ is C6, C7, C8, C9 or C10 alkyl group) are particularly preferred because their release profile can provide beneficial moisture control to the wound during the healing process.

### *5.2.3 Mixture of $R_1/R_2$=H and derivative*

A mixture of compounds according to Formula (1) or Formula (1a) can also be used for the present invention. For instance, a mixture of stereoisomers where $R_1$ and $R_2$ are both H may be used.

A compound according to Formula (1) or Formula (1a) where both $R_1$ and $R_2$ are hydrogens (H) (e.g. isosorbide) can also be used together with one or more derivatives (e.g. ester derivatives) according to Formula (1) or formula (1a). It is for instance preferred to use a mixture of isosorbide with one or more derivatives according to Formula (1a). Similarly, a derivative can be used alone or in combination with a compound where $R_1$ and $R_2$ are hydrogen (H) and/or another derivative. For example, isosorbide can be used alone or together with one or more ester derivatives of isosorbide. An ester derivative (mono- or diester) of isosorbide can be used alone or together with isosorbide and/or another ester derivative of isosorbide. The ester derivative (e.g. of isosorbide) can be used as a mixture of ester derivatives. For instance, a mixture of ester derivatives (e.g. of isosorbide) might be obtained where a mixture of fatty acids (e.g. plant derived) is used to prepare the derivative.

It has been found that a compound where $R_1$ and $R_2$ are both hydrogens (H) (e.g. isosorbide) is faster acting but shorter-lived in the wound than a derivative thereof (e.g. ester derivative of isosorbide). On the other hand, the derivatives (e.g. isosorbide ester) are longer-lived to provide a lasting therapeutic effect. While the exact mechanism remains to be elucidated, it seems that the hydrolysable groups need to be cleaved off the derivative before it can exert its therapeutic effect. The cleaving is likely performed by enzymes (such as esterases) that are upregulated in the wound bed. The different rate of action of these compounds can be put to good effect as it allows formulations to be tailored for a specific treatment. For instance, a formulation comprising the shorter-lived but fast-acting compound where $R_1$ and $R_2$ are both hydrogen (H) (e.g. isosorbide) can be used where multiple applications of the formulation are possible or necessary. Where a wound is difficult to reach or should not be disturbed (e.g. a closed wound) then the longer-lasting derivatives (e.g. ester derivatives of isosorbide) can be used.

A preferred formulation of the present invention is one comprising isosorbide and an ester derivative thereof to provide a quick but long-lasting therapeutic effect.

### *5.2.4 Pharmaceutically acceptable salts and solvates*

It is to be understood that any disclosure herein with respect to the compounds of Formulae (1) and (1a) also extends to the stereoisomers, tautomers, pharmaceutically acceptable salts, polymorphs, and solvates thereof.

"Pharmaceutically acceptable salts", as used herein, are salts that retain the desired biological activity of the parent compound and do not impart undesired toxicological effects. Pharmaceutically acceptable salt forms include various crystalline polymorphs as well as the amorphous form of the different salts. The pharmaceutically acceptable salts can be formed with metal or organic counterions and include, but are not limited to, alkali metal salts such as sodium or potassium; alkaline earth metal salts such as magnesium or calcium; and ammonium or tetraalkyl ammonium salts. The salts can be organic or inorganic in nature. Representative salts include acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate.

"Solvates", as used herein, are addition complexes in which the compound is combined with an acceptable co-solvent in some fixed proportion. Co-solvents include, but are not limited to, ethyl acetate, lauryl lactate, myristyl lactate, cetyl lactate, isopropyl myristate, methanol, ethanol, 1-propanol, isopropanol, 1-butanol, isobutanol, tert-butanol, acetone, methyl ethyl ketone, acetonitrile, benzene, toulene, xylene(s), ethylene glycol, dichloromethane, 1,2-dichloroethane, N-methylformamide, N,N-dimethylformamide, N-methylacetamide, pyridine, dioxane, and diethyl ether. The

term 'hydrate' is employed when the co-solvent is water.

### *5.3 Formulation types*

The active compounds of Formula (1) and also the preferred active compounds of Formula (1a) are preferably formulated as a composition suitable for topical administration such as a lotion, cream, gel, ointment, paste, emulsion, foam, mousse, solution, spray, dispersion, aerosol, alginates, hydrogels, and hydrocolloids sticks, bars, and films. Reference is made to "Remington: The Science and Practice of Pharmacy, 21st Ed., Chapter 39" and "Ansel's Pharmaceutical Dosage Forms and Drug Delivery Systems, 9th Ed., Section IV-10 and Section VI" for further information regarding topical formulations. Preferred topical formulations are lotions, creams, gels, and ointments.

*5.3.1 Lotions*

A lotion is a low- to medium-viscosity liquid formulation. A lotion can contain finely powdered substances that are insoluble in the dispersion medium through the use of suspending agents and dispersing agents. Alternatively, lotions can have as the dispersed phase liquid substances that are immiscible with the vehicle and are usually dispersed by means of emulsifying agents or other suitable stabilizers. In one embodiment, the lotion is in the form of an emulsion having a kinematic viscosity at 20°C of between 100 and 1000 $mm^2 \cdot s^{-1}$ (between 100 and 1000 cSt). The fluidity of lotions permits rapid and uniform application over a wide surface area. Lotions are typically intended to dry on the skin leaving a thin coat of their medicinal components on the skin's surface.

#### *5.3.2 Creams*

A cream is a viscous liquid or semi-solid emulsion of either the oil-in-water or water-in-oil type. Creams may contain emulsifying agents and/or other stabilizing agents. The oil-in-water cream bases are water-washable, and contain an oil phase, an emulsifier, and an aqueous phase. The oil phase, also called the 'internal' phase, is preferably comprised of petrolatum and a fatty alcohol such as cetyl or stearyl alcohol. The aqueous phase usually, although not necessarily, exceeds the oil phase in volume, and preferably contains a humectant. The emulsifier in a cream formulation is generally preferably a nonionic, anionic, cationic, or amphoteric surfactant. For the purposes of this invention a cream has a kinematic viscosity at 20°C of greater than 1000 $mm^2 \cdot s^{-1}$ (1000 cSt), typically in the range of 20,000-50,000 $mm^2 \cdot s^{-1}$ (20,000 to 50,000 cSt). Creams are oftentimes preferred over lotions and ointments as they are generally easier to spread and easier to remove.

The basic difference between a cream and a lotion is the viscosity, which is dependent on the amount/use of various oils and the percentage of water used to prepare the formulations. Creams are typically thicker than lotions and often use more varied oils/butters, depending upon the desired effect upon the skin. In a cream formulation, the water-base percentage is about 60-75% and the oil-base is about 20-30% of the total, with the other percentages being the active compounds or agents, emulsifier agents, preservatives and additives for a total of 100%.

#### *5.3.3 Gels*

A gel is a semisolid system containing dispersions of small or large molecules in a liquid vehicle that is rendered semisolid by the action of a thickening agent or polymeric material dissolved or suspended in the liquid vehicle. The liquid may include a lipophilic component, an hydrophilic component or both. Some emulsions may be gels or otherwise include a gel component. Some gels, however, are not emulsions because they do not contain a homogenized blend of immiscible components. Suitable gelling agents include, but are not limited to, modified celluloses, such as alginate, carboxymethyl cellulose, poly-ethylene glycol, hydroxypropyl cellulose and hydroxyethyl cellulose; carbopol homopolymers and copolymers; and combinations thereof. Suitable solvents in the liquid vehicle include, but are not limited to, diglycol monoethyl ether; alkene glycols, such as propylene glycol; dimethyl isosorbide; alcohols, such as isopropyl alcohol and ethanol; or water and buffers. The solvents are typically selected for their ability to dissolve the active compounds. Other additives, which improve the skin feel and/or emolliency of the formulation, may also be incorporated. Examples of such additives include, but are not limited, isopropyl myristate, ethyl acetate, C12-C15 alkyl benzoates, mineral oil, squalane, cyclomethicone, capric/caprylic triglycerides, and combinations thereof.

#### *5.3.4 Ointments*

An ointment is a semisolid preparation containing an ointment base and one or more of the active compounds or agents. Ointment bases may be grouped in four classes: oleaginous bases; emulsifiable-bases; emulsion bases; and water-soluble bases. Oleaginous ointment bases include, for example, vegetable oils, fats obtained from animals, and

semisolid hydrocarbons obtained from petroleum. Emulsifiable ointment bases, also known as absorbent ointment bases, contain little or no water and include, for example, hydroxystearin sulfate, anhydrous lanolin, lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin, and stearic acid. A glycerol/HEPES buffer system can be used. Preferred water-soluble ointment bases are prepared from polyethylene glycols.

*5.3.5 Hydrogels and hydrocolloids*

Hydrogels generally consist of high-molecular weight molecules that form a coherent matrix for enclosing smaller molecules and aqueous solutions. Hydrogels can be described as a two- component system of water and a three-dimensional network polymer. Examples of hydrogels include alginate, PEG, CMC, starch, pectin, gelatine, other natural gums and insoluble cross-linked polymers such as polyethylene oxide. Hydrocolloids are hydrophilic polymers, of vegetable, animal, microbial or synthetic origin, that generally contain many hydroxyl groups and may be polyelectrolytes. They are naturally present or added to control the functional properties of a material such as viscosity, including thickening and gelling, and water binding. They are advantageous for use as wound care devices because of their ability to absorb several times their weight in wound exudates. Examples of hydrocolloids include carbowax, vinyl polymers (such as polyvinyl alcohol, polyvinyl pyrrolidone, and polyvinylacetate), cellulose derivatives (such as ethyl cellulose, methyl cellulose, and carboxymethyl cellulose), and natural gums (such as guar, acacia, and pectins).

The pH of the formulation (e.g. lotion, cream, gel, ointment, hydrogel or hydrocolloid) according to the present invention is preferably between 6 and 8, more preferably between 7 and 8, more preferably between 7.3 and 7.7.

***5.4 Excipients and other additives***

When formulated for topical administration the formulation may contain one or more excipients, diluents, carriers, additives and combinations thereof that may be combined with the active compounds to enhance, facilitate or enable the production, storage, administration, delivery or effectiveness of the active compounds. The carrier medium should be dermatologically acceptable, preferably designed to reduce or avoid undue toxicity, incompatibility, instability, allergic response and the like when applied to a wound or the skin.

The CTFA International Cosmetic Ingredient Dictionary and Handbook (2004 and 2008) describes a wide variety of non-limiting ingredients that can be used in the formulations of the present invention. Examples of these ingredient classes include: one or more skin penetration enhancers (which may be surfactants, alcohols, esters, glycols or the like or any other suitable penetration enhancer), emulsifiers, moisturizers (including, e.g., emollients, humectants, film formers, occlusive agents, and agents that affect the natural moisturisation mechanisms of the skin), skin conditioning agents (e.g., aloe extracts, allantoin, bisabolol, ceramides, dimethicone, hyaluronic acid), anti-irritants (e.g. steroids and non-steroidal anti-inflammatories), anti-microbial agents (e.g. antibiotics, silver), fragrances (artificial and natural), dyes and colour ingredients (e.g., Blue 1, Blue 1 Lake, Red 40, titanium dioxide, D&C blue no. 4, D&C green no. 5, D&C orange no. 4, D&C red no. 17, D&C red no. 33, D&C violet no. 2, D&C yellow no. 10, and D&C yellow no. 11), adsorbents, lubricants, solvents, water-repellents, UV absorbers (physical and chemical absorbers such as paraminobenzoic acid ("PABA") and corresponding PABA derivatives, titanium dioxide, zinc oxide, etc.), essential oils, vitamins (e.g. A, B, C, D, E, and K), trace metals (e.g. zinc, calcium and selenium), botanical extracts (e.g. Aloe vera, chamomile, cucumber extract, Ginkgo biloba, ginseng, and rosemary), antioxidants (e.g., BHT, BHA and tocopherol), chelating agents (e.g., disodium EDTA and tetrasodium EDTA), preservatives (e.g., methylparaben and propylparaben), pH adjusters (e.g., sodium hydroxide and citric acid), absorbents (e.g., aluminum starch octenylsuccinate, kaolin, corn starch, oat starch, cyclodextrin, talcum, and zeolite), skin bleaching and lightening agents (e.g., hydroquinone and niacinamide lactate), humectants (e.g., urea and mannitol), exfoliants, waterproofing agents (e.g., magnesium/aluminum hydroxide stearate), and dipotassium glycyrrhizate). Other additional ingredients include surfactants (which may be cationic, non-ionic, anionic or polymeric), clays, anti-foaming agents, spreading agents, barriers, solubilising agents for the therapeutic agent and the like. Other exemplary compounds for different classes mentioned above can be found in the CTFA International Cosmetic Ingredient Dictionary and Handbook. For instance, Section 3 of Volume 3 (2004) lists various compounds in terms of their function (pages 2177 to 2299).

Examples of skin penetrating agents that can be used in the invention include alcohols such as dodecanol and oleyl alcohol; amines, such as isopropyl amine, diisopropyl amine, triethyl amine, triethanol amine, diisopropanolamine and ethylene diamine; carboxylic acids, such as erucic acid, oleic acid, linoleic acid, linolenic acid, and bahemic acid; esters, such as dibutyl sebacate, dibutyl phthalate, butyl benzoate, ethyl caprate and dimethyl isosorbide; and others, such as azone, N-methyl pyrollidone, bile salts and urea.

Examples of emulsifiers that can be used in the invention include, but are not limited to, methyl glucose sesquistearate, PEG-20 methyl glucoside sesquistearate, steareth-21, polyethylene glycol 20 sorbitan monostearate, polyethylene glycol 60 sorbitan monostearate, polyethylene glycol 80 sorbitan monostearate, steareth-20, ceteth-20, PEG-100 stearate, sodium stearoyl sarcosinate, hydrogenated lecithin, sodium cocoylglyceryl sulfate, sodium stearyl sulfate, sodium stearoyl lactylate, PEG-20 glyceryl monostearate, sucrose monostearate, sucrose polystearates, polyglyceryl 10 stearate, polyglcyeryl 10 myristate, steareth 10, DEA oleth 3 phosphate, DEA oleth 10 phosphate, PPG-5 ceteth 10 phosphate sodium salt, PPG-5 Ceteth 10 phosphate potassium salt, steareth-2, PEG-5 soya sterol oil, PEG-10 soya sterol oil, diethanolamine cetyl phosphate, sorbitan monostearate, diethylenglycol monostearate, glyceryl monostearate, and mixtures thereof.

Examples of emollients that can be used in the invention include, but are not limited to, almond oil, caprylic/capric triglycerides, castor oil, ceratonia extract, ceteareth-20, ceteareth-30, cetearyl alcohol, ceteth 20, cetostearyl alcohol, cetyl alcohol, cetyl stearyl alcohol, cetyl esters wax, cottonseed oil, cocoa butter, cyclomethicone, diisopropyl adipate, ethylene glycol palmitostearate, glycerin, glyceryl monooleate, glyceryl monostearate, glyceryl stearate, isopropyl myristate, isopropyl palmitate, lanolin, lanolin alcohol, hydrogenated lanolin, lecithin, liquid paraffins, linoleic acid, light mineral oil, medium-chain triglycerides, mineral oil, oleic acid, white petrolatum, polyethylene glycol, polyoxyethylene glycol fatty alcohol ethers, polyoxypropylene 15-stearyl ether, propylene glycol stearate, squalane, steareth-2 or - 100, stearic acid, soybean oil, starch, stearyl alcohol, sunflower oil, xylitol, urea and mixtures thereof.

Examples of humectants that can be used in the invention include, but are not limited to, propylene glycol, glycerin, butylene glycol, triacetin and mixtures thereof.

Examples of diluents that can be used in the invention include but are not limited to, lactose, sugar, starches, modified starches, mannitol, inorganic salts, cellulose derivatives (e.g. microcrystalline cellulose, cellulose), calcium sulfate, xylitol, lactitol and the like and mixtures thereof.

Examples of buffering agents that can be used in the invention include sodium hydroxide, potassium hydroxide, ammonium hydroxide and mixtures thereof.

Examples of chelating agents that can be used in the invention include mild agents, such as, for example, ethylenediaminetetraacetic acid (EDTA), disodium edetate and EDTA derivatives, and mixtures thereof.

Examples of preservatives include that can be used in the invention phenoxyethanol, parabens (such as methylparaben and propylparaben), propylene glycols, sorbates, urea derivatives (such as diazolindinyl urea), and mixtures thereof.

### *5.5 Additional active agents*

The active compounds of Formula (1) or Formula (1a) suitable for practising the invention (e.g. isosorbide and/or isosorbide ester derivatives) can be used in simultaneous or sequential combination with one or more other active agents ('additional active agent'). For example, when applied topically the active compound can be applied before, together with, or after an additional active agent that is normally delivered onto or through the skin for either a local or systemic effect. When applied simultaneously, the active compound of the invention according to Formula (1) or Formula (1a) (or mixtures thereof) and the additional active agent may be present in the same topical formulation (e.g. in the form of a lotion, cream, gel, or ointment).

The additional active agent includes antibiotics, analgesics, anesthetics, anti-inflammatory agents (e.g., steroidal compounds such as dexamethasone, betamethasone, prednisone, prednisolone, triamcinolone, hydrocortisone, alclometasone, amcinonide, diflorasone, etc. as well as non-steroidal antiinflammatories), anti-itch and irritation-reducing compounds (e.g., antihistamines such as diphenhydramine and psoriasis treatments), antimicrobial agents, antiseptic agents (e.g., povidone-iodine, methylbenzethonium chloride, etc.), immunomodulating agents, vitamins and the like.

Suitable antibiotics include cilastatin, clavulanic acid, folinic acid, probenecid, pyridoxine, sulbactam, dapsone, ethambutol, isoniazid, pyrazinamide, rifampin, streptomycin, capreomycin, ethionamide, para aminosalicylic acid, cycloserine, ciprofloxacin, nalidixic acid, norfloxacin, ofloxacin, imipenam, meropenem, cilistatin, cefadroxil, cefazolin, cephalexin, cephalothin, cefaclor, cefamandole, cefonicid, cefoxitin, cefuroxine, cefoperazone, cefotaxime, ceftazidime, ceftizoxime, ceftriaxone, moxalactam, cefepine, bacitracin, vancomycin, aztreonam, amoxicillin, clavulanic acid, benzathine, penicillin g, penicillin v, ampicillin, carbenicillin, indamyl, carbenicillin, mezlocillin, piperacillin, ticarcillin, cloxacillin, dicloxacillin, floxacillin, methicillin, nafcillin, oxacillin, colistmethate, polyrnixin b, trimethoprim, cotrimoxazole, mafenide, sulfadiazine, sodium sulfacetamide, sulfacytine, sulfadiazine, sulfamethoxazole, sulfapyridine, sulfasalazine, sulfisoxazole, chloramphenicol, clindamycin, spectinomycin, azithromycin, clarithromycin, erythrmoycin, erythromycin estolate, spiramycin, chlortetracycline, demeclocycline, doxycycline, minocycline, oxytetracycline, amikacin, kanamycin, neomycin, streptomycin, tobramycin, nitrofurantoin, griseofulvin, potassium iodide, fluconazole, itraconazole, ketoconazole, miconazole, clotrimazole, amphotericin b, nystatin, niclosamide, nifurtimox, piperazine, praziquantel, pyrantel pamoate, ascariasis, pinworm, thiabendazole, amodiaquine, chloroquine, hydroxychloroquine, mefloquine, primaquine, pyrimethamine, quinidine gluconate, fansidar, diloxanide furoate, melarsoprol, nifurtimox, paromomycin, pentamidine, sodium stibogluconate, suramin, metronidazole, foscarnet, 3-deoxythmidin-2-ene, dideoxycytosine, dideoxyinosine, lamivudine, azidothymidine, indinavir, ritonavir, saquinavir, acyclovir, idoxuridine, ribavirin, vidarabine, amantidine, rinantidine, pharmaceutically acceptable salts thereof, and combinations thereof. Specifically, the antibiotic can be at least one of arnphomycin, apramycin, avilamycin, azithromycin, bacitracin, bactiracin zinc, clarithromycin, clindamycin, clindamycin hydrochloride, clindamycin palmitate hydrochloride, clindamycin phosphate, dirithromycin, erythromycin, erythromycin acistrate, erthromycin estolate, erthryomycin ethlylsuccinate, erthryomycin gluceptate, erythromycin lactobionate, erthromycin propionate, erthromycin stearate, fosfomycin, fosfomycin tromethamine, josamycin, kitasamycin, lexithromycin, lincomycin, limcomycin hydrochloride, metronidazole hydrochloride, metronidazole phosphate, mirincamycin hydrochloride, paldimycin, paulomycin, pirlimycin hydrochloride, ranimycin, relomycin, roxithromycin, spectinomycin hydrochloride, spiramycin, stallimycin hydrochloride, tobramycin, vancomycin, vancomycin hydrochloride, zorbamycin, mupirocin, mupirocin calcium, and parachlorophenol.

Suitable analgesics include nonsteroidal anti-inflammatory drugs (NSAIDs) (e.g., salicylates, propionic acid derivatives, acetic acid derivatives, enolic acid derivatives, fenamic acid derivatives, COX-2 inhibitors, sulphonanilides, etc.) and opiates (e.g., morphine, codeine, thebaine, papaverine, etc.). Exemplary analgesics include acetaminophen, alfentanil hydrochloride, aminobenzoate potassium, aminobenzoate sodium, anidoxime, anileridine, anileridine hydrochloride, anilopam hydrochloride, anirolac, antipyrine, aspirin, benoxaprofen, benzydamine hydrochloride, bicifadine hydrochloride, brifentanil hydrochloride, bromadoline maleate, bromfenac sodium, buprenorphine hydrochloride, butacetin, butixirate, butorphanol, butorphanol tartrate, carbamazepine, carbaspirin calcium, carbiphene hydrochloride, carfentanil citrate, ciprefadol succinate, ciramadol, ciramadol hydrochloride, clonixeril, clonixin, codeine, codeine phosphate, codeine sulfate, conorphone hydrochloride, cyclazocine, dexoxadrol hydrochloride, dexpemedolac, dezocine, diflunisal, dihydrocodeine bitartrate, dimefadane, dipyrone, doxpicomine hydrochloride, drinidene, enadoline hydrochloride, epirizole, ergotamine tartrate, ethoxazene hydrochloride, etofenamate, eugenol, fenoprofen, fenoprofen calcium, fentanyl citrate, floctafenine, flufenisal, flunixin, flunixin meglumine, flupirtine maleate, fluproquazone, fluradoline hydrochloride, flurbiprofen, hydromorphone hydrochloride, ibufenac, indoprofen, ketazocine, ketorfanol, ketorolac tromethamine, letimide hydrochloride, levomethadyl acetate, levomethadyl acetate hydrochloride, levonantradol hydrochloride, levorphanol tartrate, lofemizole hydrochloride, lofentanil oxalate, lorcinadol, lornoxicam, magnesium salicylate, mefenamic acid, menabitan hydrochloride, meperidine hydrochloride, meptazinol hydrochloride, methadone hydrochloride, methadyl acetate, methopholine, methotrimeprazine, metkephamid acetate, mimbane hydrochloride, mirfentanil hydrochloride, molinazone, morphine sulfate, moxazocine, nabitan hydrochloride, nalbuphine hydrochloride, nalmexone hydrochloride, namoxyrate, nantradol hydrochloride, naproxen, naproxen sodium, naproxol, nefopam hydrochloride, nexeridine hydrochloride, noracymethadol hydrochloride, ocfentanil hydrochloride, octazamide, olvanil, oxetorone fumarate, oxycodone, oxycodone hydrochloride, oxycodone terephthalate, oxymorphone hydrochloride, pemedolac, pentamorphone, pentazocine, pentazocine hydrochloride, pentazocine lactate, phenazopyridine hydrochloride, phenyramidol hydrochloride, picenadol hydrochloride, pinadoline, pirfenidone, piroxicam olamine, pravadoline maleate, prodilidine hydrochloride, profadol hydrochloride, propiram fumarate, propoxyphene hydrochloride, propoxyphene napsylate, proxazole, proxazole citrate, proxorphan tartrate, pyrroliphene hydrochloride, remifentanil hydrochloride, salcolex, salicylamide, salicylate meglumine, salsalate, sodium salicylate, spiradoline mesylate, sufentanil, sufentanil citrate, talmetacin, talniflumate, talosalate, tazadolene succinate, tebufelone, tetrydamine, tifurac sodium, tilidine hydrochloride, tiopinac, tonazocine mesylate, tramadol hydrochloride, trefentanil hydrochloride, trolamine, veradoline hydrochloride, verilopam hydrochloride, volazocine, xorphanol mesylate, xylazine hydrochloride, zomepirac sodium, and zucapsaicin.

Suitable anesthetics include aliflurane, articaine, benoxinate hydrochloride, benzocaine, biphenamine hydrochloride, bupivacaine hydrochloride, butamben, butamben picrate, chloroprocaine hydrochloride, cocaine, cocaine hydrochloride, cyclopropane, desflurane, dexivacaine, diamocaine cyclamate, dibucaine, dibucaine hydrochloride, dyclonine hydrochloride, enflurane, ether, ethyl chloride, etidocaine, etoxadrol hydrochloride, euprocin hydrochloride, fluroxene, halothane, isobutamben, isoflurane, ketamine hydrochloride, levobupivacaine, levoxadrol hydrochloride, lidocaine, lidocaine hydrochloride, mepivacaine hydrochloride, methohexital sodium, methoxyflurane, midazolam hydrochloride, midazolam maleate, minaxolone, norflurane, octodrine, oxethazaine, phencyclidine hydrochloride, pramoxine hydrochloride, prilocaine hydrochloride, procaine hydrochloride, propanidid, proparacaine hydrochloride, propofol, propoxycaine hydrochloride, pyrrocaine, risocaine, rodocaine, roflurane, ropivacaine, salicyl alcohol, sevoflurane, teflurane, tetracaine, tetracaine hydrochloride, thiamylal, thiamylal sodium, thiopental sodium, tiletamine hydrochloride, and zolamine hydrochloride.

Suitable immunomodulating agents, include azathioprine, 6- mercaptopurine, cyclosporin, methotrexate, interferon $\alpha$ IIb, autologous granulocyte macrophagecolony stimulating factor, APC 8015 (Provenge), cancer vaccines, antisense oligonucleotides, bacillus of Calmette-Guerin (BCG), and the like.

Other additional active agents include salicylic acid, cholesterol benzoate, retinyl acetate, ammonium bituminosulfonate (ichthammol), chloorhexidine, and sucrose octasulphate.

When the wound to be treated is an acute wound then the formulations of the present invention preferably do not contain a mono or di-nitrate derivative of Formula (1) (e.g. isosorbide mono/di-nitrate) because the release of NO from these compounds will act on the vascular endothelium which will dilate and possibly prolong or cause bleeding in acute wounds. When the wound to be treated is a chronic wound then the use of a combination of the compound of Formula (1), or preferably Formula (1a), with a nitric oxide donor such as isosorbide mono- or di-nitrate can be valuable.

The release of isosorbide from the compound of Formula (1) or Formula (1a) will reduce scarring while the release of NO from isosorbide mono- or di-nitrate will stimulate angiogenesis. When the wound to be treated is an acute wound then the formulations of the present invention preferably do not contain a nitric oxide donor such as isosorbide mono- or dinitrate.

## 6. Amount of active compound and dosing regimen

### *6.1 Amount of active compound*

The topical formulations of the present invention typically contain 1 to 10,000 μM, preferably from 30 to 3000 μM of the active compound according to Formula (1) or Formula (1a). Alternatively or additionally, the topical formulations of the invention contain from 0.0001 to 100 wt.%, preferably 0.0005 to 10 wt.%, more preferably 0.001 to 2 wt.%, even more preferably 0.01 to 0.1 wt.% of the active compound according to Formula (1) or Formula (1a). If a mixture of two or more of the active compounds of Formula (1) and/or a mixture of compounds of Formula (1a) is present, the above ranges apply to the sum of the weight contents of the active compounds that are present.

The active compound (e.g. isosorbide and/or isosorbide ester derivative) should be used in a therapeutically effective amount. A "therapeutically effective amount" means the amount of the active compound (or mixture thereof) that, when administered to a patient for treating a wound or reducing scarring, is sufficient to induce a medically beneficial effect. The therapeutically effective amount may vary depending on the active compound, the specific nature of the wound and the age, weight or other characteristic of the patient to be treated.

When considered as a function of area, it is preferable to apply the compound evenly over a surface to be treated so as to arrive at an amount of 1 to 10,000 $mg/m^2$, preferably 10 to 1,000 $mg/m^2$ and more preferably 50 to 500 $mg/m^2$.

### *6.2 Dosing regimen*

The dosage may be delivered by a single administration or by multiple applications as needed to achieve the most effective results. Dosing can continue for as long as is medically indicated, which will depend on the severity of the wound.

It is preferable to treat a wound as quickly as possible in view of increased skin permeability immediately after wounding. Low molecular weight (< 500 Da) and moderately lipophilic agents (logP 1-3) can surmount the horny barrier layer and gain access to viable epidermis, dermis and blood vessels to a low, yet relevant extent (Bätz et al. 2012).

When treating acute wounds, it is preferable that the compounds according to Formula (1) (or a formulation comprising these compounds) be applied to the wound (or closed wound) within 1 day of the wound being formed. It is more preferable to apply the compounds within 6 hours of wound formation, even more preferable within 1 hour of wound formation.

For wounds created during surgery the compounds according to Formula (1) (or a formulation comprising these compounds) are preferably applied to the open wound and again on the closed wound on a regular basis for at least one week. An example of a regular basis is one, two or three separate applications per day.

The present invention is now illustrated in greater detail by way of the following Examples, but it should be understood that the present invention is not deemed to be limited thereto.

## 7. Examples

### 7.1 Preparation of compositions

Composition 1: Glycerol 50% v/v containing 20 mM HEPES and 150 mM NaCl.

Composition 2: Glycerol 50% v/v containing 20 mM HEPES, 150 mM NaCl and 30μM isosorbide.

Composition 3: Glycerol 50% v/v containing 20 mM HEPES, 150 mM NaCl and 300μM isosorbide.

Composition 4: Glycerol 50% v/v containing 20 mM HEPES, 150 mM NaCl and 300μM isosorbide.

Composition 5: Glycerol 50% v/v containing 20 mM HEPES, 150 mM NaCl and 30μM Polysorb.

All of the above compositions had a pH between 7.5 and 7.6. Additional ingredients that are common in topical formulations have been kept to a minimum so as to examine the effect that compounds according to Formula (1) have on the wound healing process. Further topical formulations are described below.

**7.2 Animal preparation and treatment**

Mouse models have contributed significantly to an understanding of skin biology and disease and so are an appropriate model for testing the compounds and formulations of the invention. The mouse model used here can be considered one of the simplest and standard murine models for investigating scar formation and wound healing.

***7.2.1 Anaesthesia and surgery***

Mice were weighed and then anaesthetized with 5% isoflurane in air for induction and then administered 2.5-3% of isoflurane in air for the remainder of the procedure. The animals were administered Tramal (Tramadol) intraperitoneally for analgesia at a 0.1mg/kg dose. Before surgery the back of the mice was shaved and disinfected with isobetadine soap, rinsed with water and then swabbed with iodium alcohol.

***7.2.2 Excisional model (wound left open):***

The back of the mice was shaved and four full-thickness skin wounds were performed by cutting the folded skin on the back of the mice with a 7.5 mm x 3 mm elliptical biopsy punch (Elliptical Excisional Instrument, Acuderm Inc.). The skin on the back was raised and folded at the level of the vertebral column by using the thumb and the index finger. The folded skin was then placed on a wood tongue depressor with the animal laying on one side and two wounds were performed through the folded skin by using a elliptical biopsy punch (Elliptical Excisional Instrument, Acuderm Inc.), resulting in four wounds on the back skin: two on the right of the vertebral column and two on the left (Figure 9). The epidermis, the dermis and the fat tissue in the wound was carefully removed without touching other tissues. The wounds were then immediately treated with topical application of either the hydrogel or the ointment (glycerol + HEPES buffer) formulation containing the active substance (T1 and T2) or vehicle (Tv) and one wound was (Ts) left untreated (Figure 9). The position of the wounds was randomised according to a list. The wound site was then covered with OP-Film (Somed International, the Netherlands) (Figure 9). The borders were then fixed with cyanoacrylate glue (Loctite).

***7.2.3 Incisional model (wound subsequently closed):***

This model is identical to the excisional model above except that the wounds were closed with three sutures.

***7.2.4 Post-operative care***

The mice were allowed to recover from the surgical preparation in a clean cage with fresh bedding and under a heating lamp. To avoid possible contamination the mice were placed in individual cages containing a cellulose bed that was changed every 24 hours. Single caging avoids other mice from detaching the adhesive patches.

***7.2.5 Post-operative treatment***

The treatment was repeated at the day 1, 2, 3 and 4 post-surgery for a total of 5 treatments in the first 5 days of healing. At each time point the mice were briefly anaesthetized with isoflurane and a new dose of ointment was injected through the film onto the wound, after which the animal was left to recover. At day 5 all films were removed.

***7.2.6 Tissue harvest***

After 14 days the mice were sacrificed under isoflurane anaesthesia by cervical dislocation to harvest the regenerated tissue. At sacrifice the mice were weighed and a gross necropsy was performed during which the following tissues are harvested:

Plasma: stored at -20°C
Liver: fixed (4% PFA in PBS @ 4°C 24h)
Kidney: fixed (4% PFA in PBS @ 4°C 24h)
Spleen: fixed (4% PFA in PBS @ 4°C 24h)

Skin samples were also taken. The back of the mouse was carefully shaved paying careful attention to not touch the wound area. Three deep cuts in the back skin were made using a surgical blade to harvest two skin strips containing the four residual wounds (two wounds per strip). A scissors was used to harvest a square of skin containing the residual wound.

The skin wounds were split into two parts perpendicular to the direction of the incision. One part was imbedded in OCT and frozen on dry ice and the last one fixed in 4% paraformaldehyde for embedding in paraffin.

The segment for cryosectioning was prepared as follows. The mold was partially filled with the OCT medium, on top of which the sample was placed. Hereafter the remainder of the mold was filled with OCT. Care was taken not to have air bubbles close to the samples and a mark was made to identify the side of the wound. The sample was frozen by placing the mold on dry ice. The samples were stored at -20°C until shipment.

The other part of the section was fixed for further processing. The sample was placed between two pieces of paper on which a marking was made identifying the wound side of the sample. The sample was then placed in a cassette and a polyurethane sponge was placed on top. The cassette was closed and placed in a 4% ice-cold paraformaldehyde solution for 24 hrs after which they were transferred to 70% ethanol solution and paraffin-embedded for sectioning.

### 7.3 Measuring the extent of scarring

The extent of scarring was measured in accordance with the procedure set out by Khorasani et al (2011).

H&E staining photographs were captured on an Olympus Slide Scanner VS120-L100 (Olympus America Inc., Center Valley, PA) equipped with Camera Pike F505 C Color at 20× magnification. Image analysis was performed using the NIH program ImageJ, available as shareware from the NIH website

Dermal thickness was defined as the distance from the epidermal-dermal junction down to the panniculus carnosus visualized by H&E stain. Using ImageJ, dermal thickness measurements were obtained on 40× light microscope images by drawing a line normal to the average orientation of the epidermal-dermal and dermal-subcutaneous tissue demarcations. Four dermal thickness measurements were taken per sample-two adjacent to the wound site at 50 μm on either side, and two at a farther distance of 700 μm on either side of the wound.

Fibrotic scar tissue was outlined using the freeform outline tool in ImageJ to produce a pixel-based area measurement that could then be converted to square micrometers. Like the dermal thickness measurements, scar area measurements were performed extended to the panniculus carnosus. Scar size is determined by the Scar Index, which is calculated by dividing the scar area (A, in $\mu m^2$) by the corresponding average dermal thickness ($T_{avg}$, in μm):

$$\text{Scar Index} = A/T_{avg}$$

Collagen content and organisation were assessed as published by De Visscher et al. (2007, 2008). In short, sections are stained with picrosirius red and then imaged at 20x. The imaging is done with a mosaic of overlays of bright-field and polarised light images. A semi-automatic analysis tool was developed allowing assessment of the surfaces covered by collagen and organised collagen. From this the collagen content and organisation can be measured according to the following method.

The total area (At) of the region of interest (ROI) and the area of the red stained material within that area (Ar) on the bright-field image. On the polarized light image, the area of the red illuminated bundles (Ab) is measured. The collagen density and organized collagen are calculated by the following formulae:

$$\text{(1) Collagen density: } (Ar/At) \times 100$$

$$\text{(2) Organized collagen: } (Ab/Ar) \times 100$$

Both are expressed as a percentage: collagen density as a percentage of the total area and organized collagen as a percentage of the collagen area.

### 7.4 Measuring the extent of tissue regeneration

Histological slides were examined to determine the presence of tissue structures such as sebaceous glands and hair follicles in the scar tissue. Sebaceous glands and hair follicles were identified according to their morphologies in slides stained with hematoxylin and eosin.

### 7.5 Results

Histological analysis was performed and the scar index measured (Table 1) according to the protocol described above (see chapter 7.3). A statistically significant decrease compared (1-way ANOVA with Dunnet's comparison to Vehicle-treated group, $p<0.05$) in the scar index was observed when wounds were treated with isosorbide 30 μM (Composition 2), a scar index of 517.33 μm ± 247.92 μm was observed. As expected, a severe scar formation was observed in untreated and in vehicle-treated wounds where a high scar index corresponding to 1152 μm ± 621.85 μm and 819 μm ± 285.89 μm, respectively was observed (Table 1).

Table 1:

| Composition | Average of Scar Index | 95% CI |
|---|---|---|
| Untreated | 1152.45 | 430.92 |
| Composition 1 (Vehicle) | 819.36 | 177.20 |
| Composition 2 (Isosorbide 30μM) | 517.33 | 191.97 |
| Composition 3 (Isosorbide 300μm) | 629.54 | 345.47 |
| Composition 4 (Isosorbide 3000μm) | 703.39 | 265.60 |

Collagen content was measured by image analysis in order to quantify the amount of collagen in the wounds (Table 2). The lowest collagen content was observed in untreated wounds (47.77 ± 14.65).

Table 2:

| Composition | Average of Collagen Content in the wound (%) | 95%CI of Collagen Content in the wound (%) |
|---|---|---|
| Untreated | 47.77 | 10.15 |
| Composition 1 (Vehicle) | 56.80 | 4.60 |
| Composition 2 (Isosorbide 30μM) | 60.34 | 8.58 |
| Composition 3 (Isosorbide 300μm) | 60.21 | 13.64 |
| Composition 4 (Isosorbide 3000μm) | 57.17 | 19.48 |

Collagen organisation showed a similar trend with increased values upon administration of isosorbide.

Table 3:

| Composition | Average of Collagen organisation in the wound (%) | 95%CI of Collagen organisation in the wound (%) |
|---|---|---|
| Untreated | 12.46 | 6.54 |
| Composition 1 (Vehicle) | 9.52 | 3.05 |
| Composition 2 (Isosorbide 30μM) | 20.74 | 14.54 |
| Composition 3 (Isosorbide 300μm) | 16.76 | 20.26 |
| Composition 4 (Isosorbide 3000μm) | 16.21 | 9.03 |

### 7.6 Further topical formulations

The following topical formulations can also be used in methods for treating wounds according to the invention.

| | |
|---|---|
| **Cream formulation (ingredient and wt.%)** | |
| Isosorbide | 0.15% |
| Stearic acid | 6% |
| Stearyl alcohol | 4% |
| Cetyl alcohol | 2.5% |
| Glycerin | 12% |
| Sodium lauryl sulfate | 1% |
| Propylparaben | 0.05% |
| Methylparaben | 0.25% |
| Disodium EDTA | 0.055% |
| Distilled water | QS |
| **Ointment formulation (ingredient and weight)** | |
| Isosorbide di(octanoic acid)ester | 1 g |
| Cholesterol | 32 g |
| Stearyl Alcohol | 33 g |
| White Wax | 75 g |
| White Petrolatum | 859 g |
| **Gel formulation (ingredient and wt.%)** | |
| Isosorbide | 0.5% |
| Isosorbide di(octanoic acid)ester | 0.5% |
| Methylparaben | 0.10% |
| Propylparaben | 0.05% |
| Carbomer 934P NF | 1.5% |
| Sodium Hydroxide | QS pH 7 |
| Purified Water USP | QS 100% |
| **Lotion formulation (ingredient and weight)** | |
| Isosorbide | 0.01 g |
| Castor oil | 4.5 g |
| Petrolatum | 1.2 g |
| PEG fatty alcohol ether | 2.7 g |
| Butylated hydroxytoluene (BHT) | 0.02 g |
| Propylene glycol | 13 g |
| Purified Water USP | QS 100 g |
| **Hydrogel formulation (ingredient and wt.%)** | |
| Isosorbide | 0.5% |
| Isosorbide di(octanoic acid)ester | 0.25% |
| Isosorbide di(decanoic acid)ester | 0.25% |
| Propylene Glycol | 13.5% |
| Triacetin (Glycerol Triacetate) | 5.0% |
| Ethanol | 17.6% |
| Lauryl Alcohol | 0.8% |
| Nonanol (Nonyl Alcohol) | 0.8% |
| Pure Water | 20.3% |
| Hydroxyethyl Cellulose (Mn: 250,000) | 6.0% |
| Polyvinyl Pyrrolidine (Collidon 90™) | 10.0% |
| 25% PVA Aqueous Solution (Degree of Polymerization: 500-2,000) | 25.0% |

The formulations above should not be considered as a limitation upon the formulations that can be used according to the invention but merely as being illustrative and representative thereof. Methods for preparing these

formulations are known, or will be apparent, to those skilled in the art. See, e.g., "Remington: The Science and Practice of Pharmacy, 21st Ed., Chapter 39" and "Ansel's Pharmaceutical Dosage Forms and Drug Delivery Systems, 9th Ed., Section IV-10 and Section VI". Reference is also made to the CTFA International Cosmetic Ingredient Dictionary and Handbook (2004 and 2008) which describes a wide variety of ingredients (such as those above as well as possible replacements or additional ingredients) that can be used in the formulations of the present invention.

**8. Industrial applicability**

The present invention provides methods, compound and formulations that are useful in the treatment of wounds, in particular the reduction of scar tissue formation and the regeneration of normal tissue after wounding. The methods, compound and formulations disclosed herein will provide therapeutic benefit to subjects suffering from wounds.

**9. Cited References**

Archer, H G, S Barnett, S Irving, K R Middleton, and D V Seal. 1990. "A controlled model of moist wound healing: comparison between semi-permeable film, antiseptics and sugar paste." Journal of experimental pathology (Oxford, England) 71(2):155-70. Retrieved (http://www.pubmedcentral.nih.gov/articlerender.fcgi?artid=1998714&tool=pmc entrez&rendertype=abstract).

Bätz, Franzisca Marie et al. 2012. "Esterase activity in excised and reconstructed human skin - Biotransformation of prednicarbate and the model dye fluorescein diacetate." European journal of pharmaceutics and biopharmaceutics : official journal of Arbeitsgemeinschaft fur Pharmazeutische Verfahrenstechnik e. V. Retrieved May 21, 2013 (http://www.ncbi.nlm.nih.gov/pubmed/23201050).

Chirife, J, G Scarmato, and L Herszage. 1982. "Scientific basis for use of granulated sugar in treatment of infected wounds." Lancet 1(8271):560-1. Retrieved January 24, 2013 (http://www.ncbi.nlm.nih.gov/pubmed/6120410).

Chirife, Jorge, L Herszage, A Joseph, and E S Kohn. 1983. "In vitro study of bacterial growth inhibition in concentrated sugar solutions: microbiological basis for the use of sugar in treating infected wounds." Antimicrobial agents and chemotherapy 23(5):766-73. Retrieved January 24, 2013 (http://aac.asm.org/content/23/5/766.short).

Forrest, R D. 1982a. "Development of wound therapy from the Dark Ages to the present." Journal of the Royal Society of Medicine 75(4):268-73. Retrieved (http://www.pubmedcentral.nih.gov/articlerender.fcgi?artid=1437659&tool=pmc entrez&rendertype=abstract).

Forrest, R D. 1982b. "Early history of wound treatment." Journal of the Royal Society of Medicine 75(3):198-205. Retrieved (http://www.pubmedcentral.nih.gov/articlerender.fcgi?artid=1437561 &tool=pmc entrez&rendertype=abstract).

Kaufman, T, E H Eichenlaub, M F Angel, M Levin, and J W Futrell. 1985. "Topical acidification promotes healing of experimental deep partial thickness skin burns: a randomized double-blind preliminary study." Burns, including thermal injury 12(2):84-90. Retrieved January 24, 2013 (http://www.sciencedirect.com/science/article/pii/0305417985900324).

Khorasani, Hooman et al. 2011. "A quantitative approach to scar analysis." The American journal of pathology 178(2):621-8. Retrieved December 31, 2012 (http://www.pubmedcentral.nih.gov/articlerender.fcgi?artid=3070584&tool=pmc entrez&rendertype=abstract).

Knutson, R A, L A Merbitz, M A Creekmore, and H G Snipes. 1981. "Use of sugar and povidone-iodine to enhance wound healing: five year's experience." Southern medical journal 74(11):1329-35. Retrieved January 24, 2013 (http://www.ncbi.nlm.nih.gov/pubmed/7302631).

Kössi, J a, T O Ekfors, V Aaltonen, and M Laato. 2000. "Sucrose has no beneficial effects on wound healing in rats." The European journal of surgery = Acta chirurgica 166(10):818-22. Retrieved (http://www.ncbi.nlm.nih.gov/pubmed/11071171).

Leveen, H H et al. 1973. "Chemical acidification of wounds. An adjuvant to healing and the unfavorable action of alkalinity and ammonia." Annals of surgery 178(6):745-53. Retrieved (http://www.pubmedcentral.nih.gov/articlerender.fcgi?artid=1355839&tool=pmc entrez&rendertype=abstract).

Miyagawa, Maiko et al. 2009. "Endolymphatic hydrops and therapeutic effects are visualized in 'atypical' Meniere's disease." Acta oto-laryngologica 129(11):1326-9. Retrieved January 24, 2013 (http://www.ncbi.nlm.nih.gov/pubmed/19863332).

Nozawa, I et al. 1995. "Efficacy of long-term administration of isosorbide for Ménière's disease." ORL; journal for oto-rhino-laryngology and its related specialties 57(3):135-40. Retrieved January 24, 2013 (http://www.ncbi.nlm.nih.gov/pubmed/7603692).

Singer, A J, and R A Clark. 1999. "Cutaneous wound healing." The New England journal of medicine 341(10):738-46. Retrieved May 15, 2013 (http://www.ncbi.nlm.nih.gov/pubmed/10471461).

Trouillet, J L et al. 1985. "Use of granulated sugar in treatment of open mediastinitis after cardiac surgery." Lancet 2(8448):180-4. Retrieved January 24, 2013 (http://www.ncbi.nlm.nih.gov/pubmed/2862372).

De Visscher, Geofrey et al. 2007. "In vivo cellularization of a cross-linked matrix by intraperitoneal implantation: a new tool in heart valve tissue engineering." European heart journal 28(11):1389-96. Retrieved January 16, 2013 (http://www.ncbi.nlm.nih.gov/pubmed/17244642).

De Visscher, Geofrey et al. 2008. "Functional and biomechanical evaluation of a completely recellularized stentless pulmonary bioprosthesis in sheep." The Journal of thoracic and cardiovascular surgery 135(2):395-404. Retrieved January 16, 2013 (http://www.ncbi.nlm.nih.gov/pubmed/18242275).

**Claims**

1. A compound according to Formula (1), or a pharmaceutically acceptable salt or solvate thereof, for use in a method of treating a wound, wherein the method comprises a step of topically administering the compound to the wound of a patient:

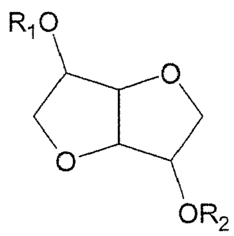

Formula (1)

wherein $R_1$ and $R_2$ may be the same or different and are selected from the group consisting of hydrogen (H) and a physiologically hydrolyzable chemical group consisting of alkylcarbonyl, alkenylcarbonyl arylcarbonyl, heteroarylcarbonyl, alkoxycarbonyl, aryloxycarbonyl, heteroaryloxycarbonyl, alkylether, alkenylether, arylether, and heteroarylether groups wherein the alkyl moiety consists of unsubstituted or substituted, straight-chain or branched-chain and cyclic alkyl groups having 2-22 carbon atoms, wherein the alkenyl moiety consists of unsubstituted and substituted, straight-chain or branched-chain and cyclic alkenyl groups having 2-22 carbon atoms, wherein the aryl moiety consists of unsubstituted and substituted phenyl, and phenalkyl groups wherein the alkyl moiety contains 1-3 carbon atoms and the phenyl moiety is unsubstituted or substituted, and the heteroaryl moiety is an aromatic 5- or 6-membered heterocyclic ring containing one or two heteroatoms selected from the group consisting of nitrogen, oxygen, and sulphur.

2. A topical formulation for use in a method of treating a wound, wherein the method comprises a step of administering the topical formulation to the wound of a patient, and wherein the formulation comprises one or more compounds

according to Formula (1), or pharmaceutically acceptable salts or solvates thereof:

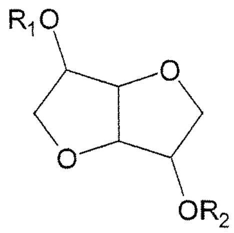

Formula (1)

wherein $R_1$ and $R_2$ may be the same or different and are selected from the group consisting of hydrogen (H) and a physiologically hydrolyzable chemical group consisting of alkylcarbonyl, alkenylcarbonyl arylcarbonyl, heteroarylcarbonyl, alkoxycarbonyl, aryloxycarbonyl, heteroaryloxycarbonyl, alkylether, alkenylether, arylether, and heteroarylether groups wherein the alkyl moiety consists of unsubstituted or substituted, straight-chain or branched-chain and cyclic alkyl groups having 2-22 carbon atoms, wherein the alkenyl moiety consists of unsubstituted and substituted, straight-chain or branched-chain and cyclic alkenyl groups having 2-22 carbon atoms, wherein the aryl moiety consists of unsubstituted and substituted phenyl, and phenalkyl groups wherein the alkyl moiety contains 1-3 carbon atoms and the phenyl moiety is unsubstituted or substituted, and the heteroaryl moiety is an aromatic 5- or 6-membered heterocyclic ring containing one or two heteroatoms selected from the group consisting of nitrogen, oxygen, and sulphur.

3. The compound for use according to claim 1 or the topical formulation for use according to claim 2, wherein the compound according to Formula (1) is a compound according to Formula (1a):

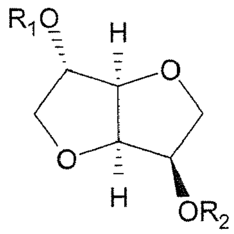

Formula (1a)

wherein $R_1$ and $R_2$ may be the same or different and have the same meaning as defined for claims 1 and 2.

4. The compound or topical formulation for use according to claims 1 to 3, wherein treating a wound means reducing scar formation, regenerating normal tissue and/or promoting the generation of normal tissue/skin structures.

5. The compound or topical formulation for use according to claims 1 to 4, wherein $R_1$ and $R_2$ are selected from the group consisting of H or $-COR_3$, wherein $R_3$ is a C2 to C22 hydrocarbon group, preferably a linear C6, C7, C8, C9 or C10 alkyl group.

6. The topical formulation for use according to any one of claims 2 to 5, comprising two or more compounds according to Formula (1) wherein for one of those compounds both $R_1$ and $R_2$ are H.

7. The topical formulation for use according to any one of claims 2 to 6, comprising two or more compounds according to Formula (1) wherein for one of those compounds both $R_1$ and $R_2$ are $-COR_3$ wherein $R_3$ is a linear C6, C7, C8, C9 or C10 alkyl group.

8. The topical formulation for use according to any one of claims 2 to 7, comprising two or more compounds according to Formula (1) wherein for one of those compounds both $R_1$ and $R_2$ are H and for a second of those compounds

both $R_1$ and $R_2$ are $-COR_3$ wherein $R_3$ is a linear C6, C7, C8, C9 or C10 alkyl group.

**9.** The topical formulation for use according to any one of claims 2 to 8, wherein the formulation is a lotion, cream, gel, or ointment.

**10.** The topical formulation for use according to any one of claims 2 to 9, further comprising a skin penetration agent.

**11.** The topical formulation for use according to any one of claims 2 to 10, wherein the wound is an open or closed acute wound and the method comprises applying the formulation to the wound bed within 1 hour of the wound being formed.

**12.** A composition comprising isosorbide and at least one compound according to Formula (1), preferably Formula (1a), or a pharmaceutically acceptable salt or solvate thereof:

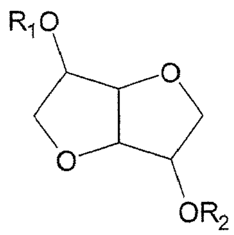

Formula (1)

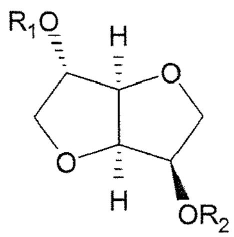

Formula (1a)

wherein $R_1$ and $R_2$ are the same or different and are selected from the group consisting of hydrogen (H) and a physiologically hydrolyzable chemical group consisting of alkylcarbonyl, alkenylcarbonyl arylcarbonyl, heteroarylcarbonyl, alkoxycarbonyl, aryloxycarbonyl and heteroaryloxycarbonyl, alkylether, alkenylether, arylether, and heteroarylether groups wherein the alkyl moiety consists of unsubstituted or substituted, straight-chain or branched-chain and cyclic alkyl groups having 2-22 carbon atoms, wherein the alkenyl moiety consists of unsubstituted and substituted, straight-chain or branched-chain and cyclic alkenyl groups having 2-22 carbon atoms, wherein the aryl moiety consists of unsubstituted and substituted phenyl, and phenalkyl groups wherein the alkyl moiety contains 1-3 carbon atoms and the phenyl moiety is unsubstituted or substituted, and the heteroaryl moiety is an aromatic 5- or 6-membered heterocyclic ring containing one or two heteroatoms selected from the group consisting of nitrogen, oxygen, and sulphur, provided that at least one of $R_1$ and $R_2$ is a physiologically hydrolyzable chemical group.

**13.** The composition according to claim 12, wherein $R_1$ and $R_2$ are the same or different and are selected from the group consisting of hydrogen (H) and $-COR_3$ provided that at least one of $R_1$ and $R_2$ is $-COR_3$, wherein $R_3$ is a linear or branched C2 to C22 alkyl.

**14.** The composition according to claim 13, wherein both $R_1$ and $R_2$ are $-COR_3$.

**15.** The composition according to claim 13 or 14, wherein $R_3$ is a linear C6, C7, C8, C9 or C10 alkyl group.

## FIGURES

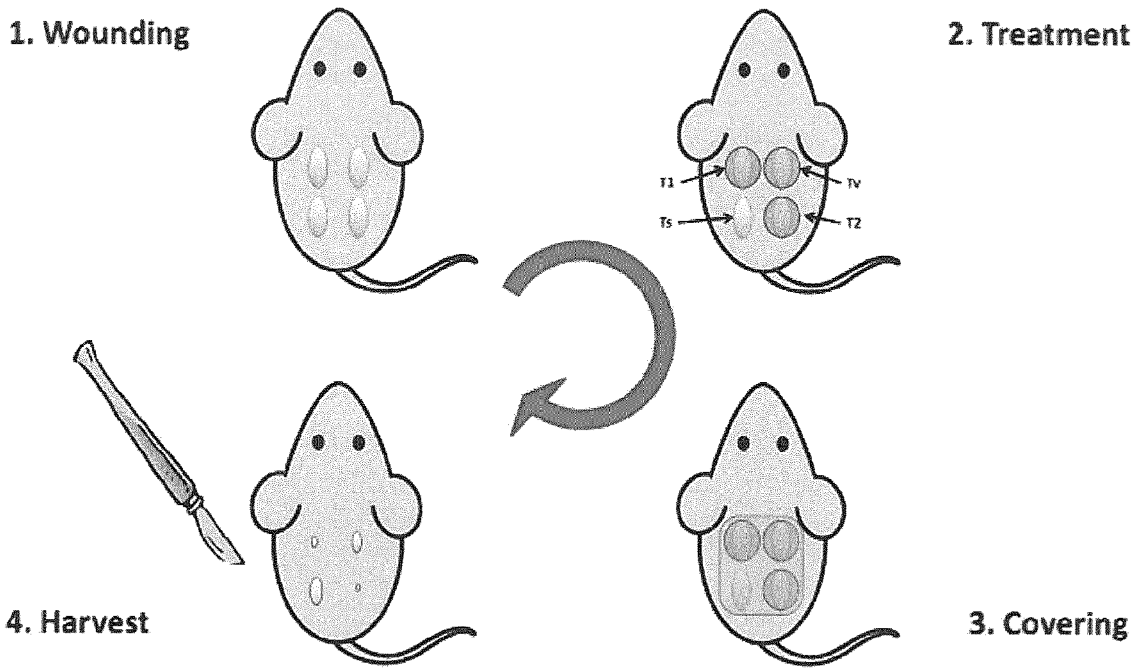


FIG. 1

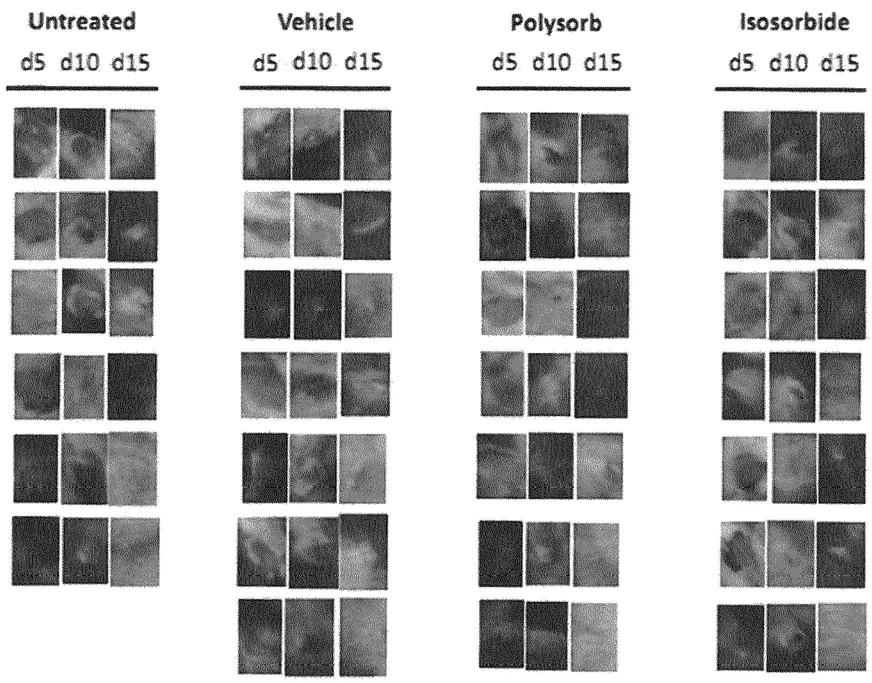
Untreated
d5 d10 d15
Vehicle
d5 d10 d15
Polysorb
d5 d10 d15
Isosorbide
d5 d10 d15

FIG. 2

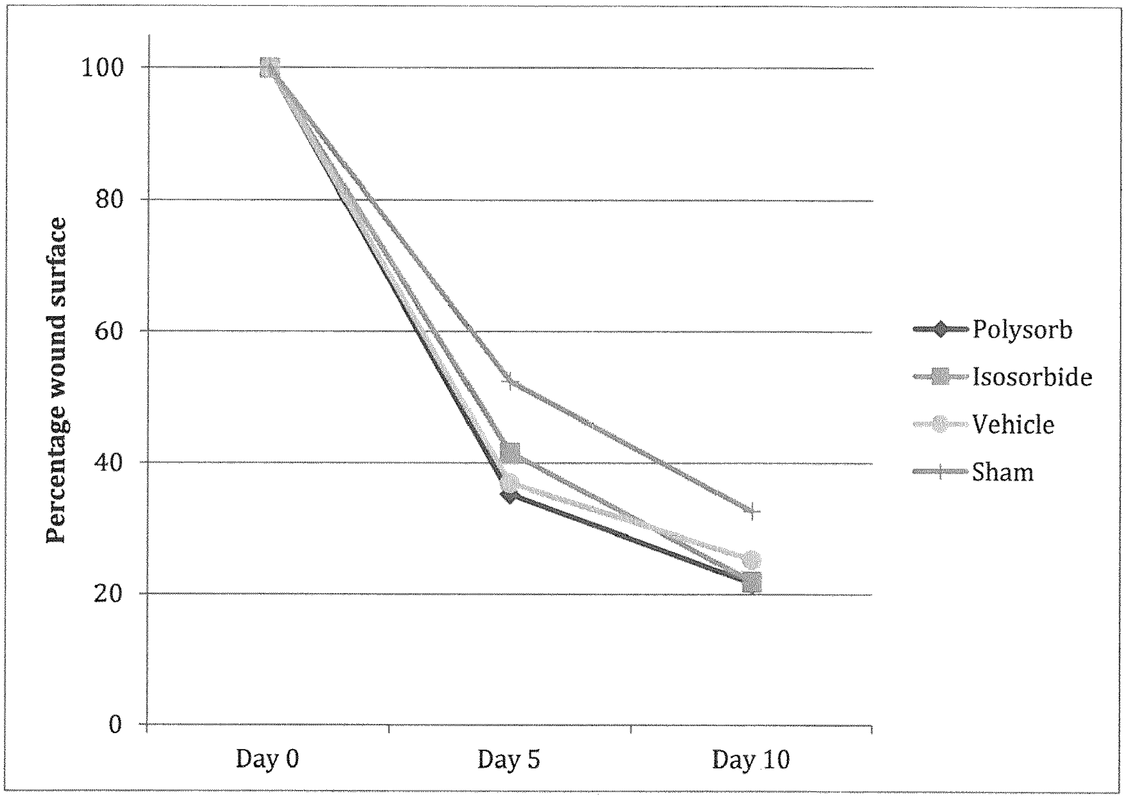
100
80
60
40
20
0
Percentage wound surface
Day 0
Day 5
Day 10
Polysorb
Isosorbide
Vehicle
Sham

FIG. 3

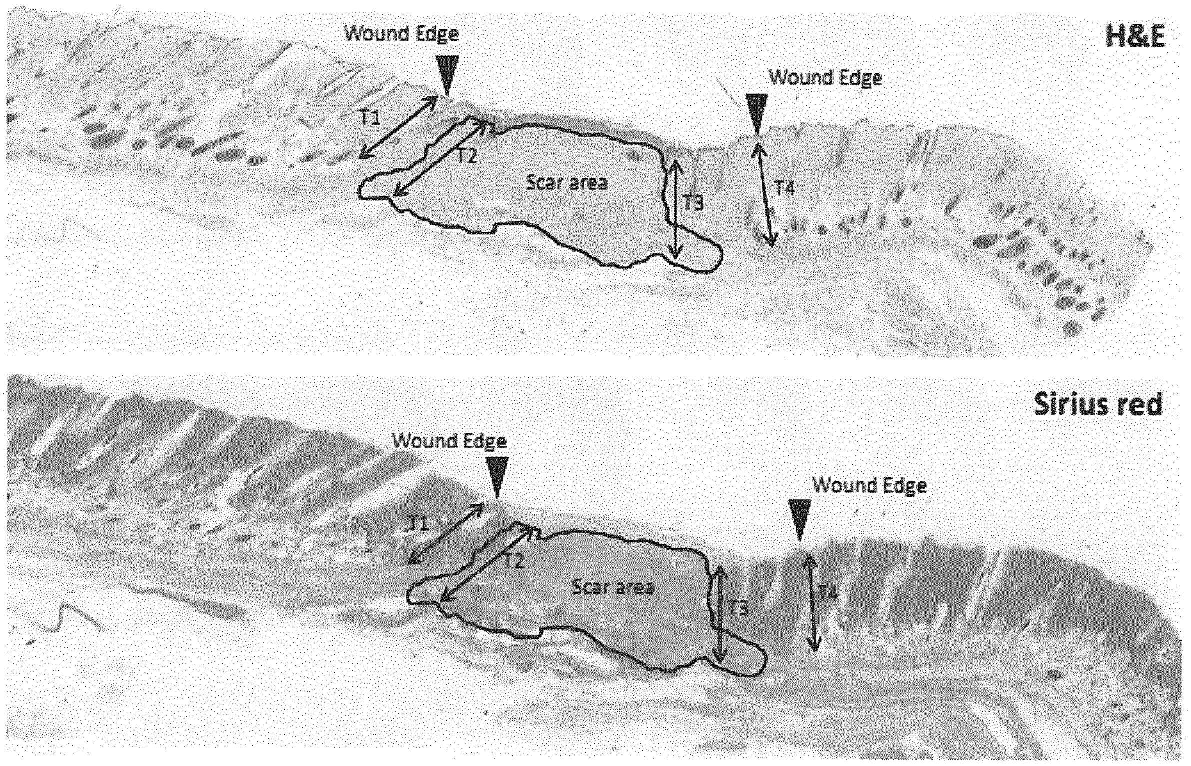
H&E
Wound Edge
Wound Edge
T1
T2
Scar area
T3
T4
Sirius red
Wound Edge
Wound Edge
T1
T2
Scar area
T3
T4

FIG. 4

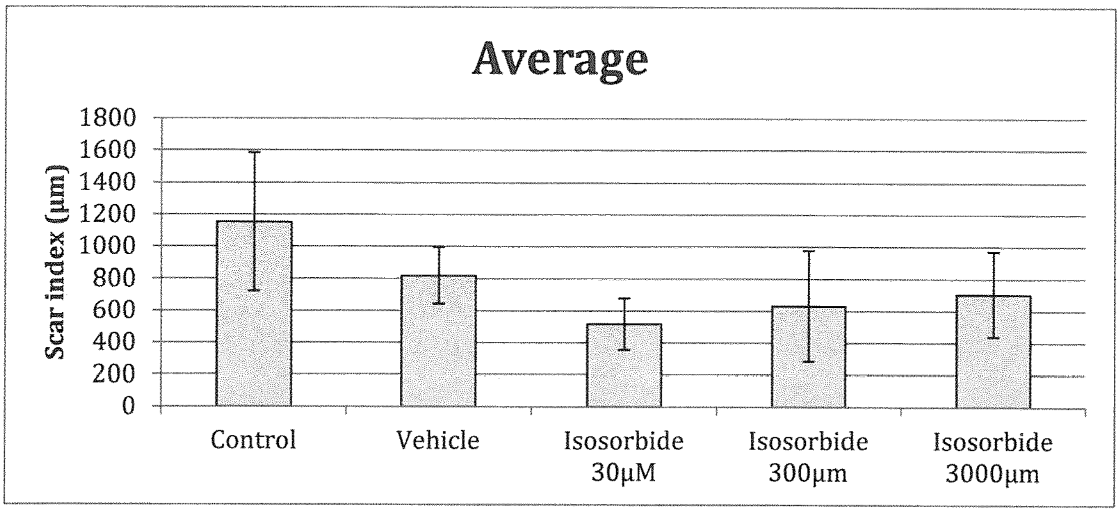
Average
Scar index (µm)
1800
1600
1400
1200
1000
800
600
400
200
0
Control
Vehicle
Isosorbide 30µM
Isosorbide 300µm
Isosorbide 3000µm

FIG. 5

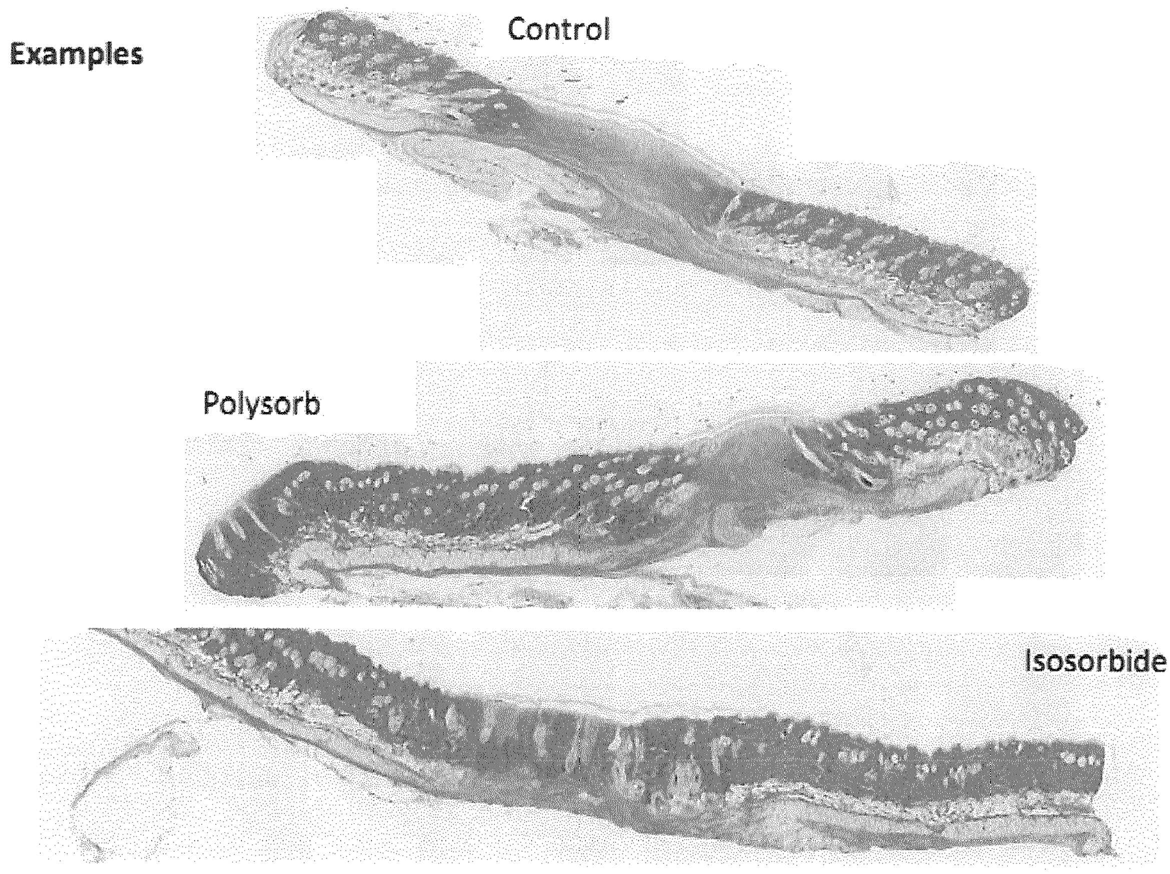


FIG. 6

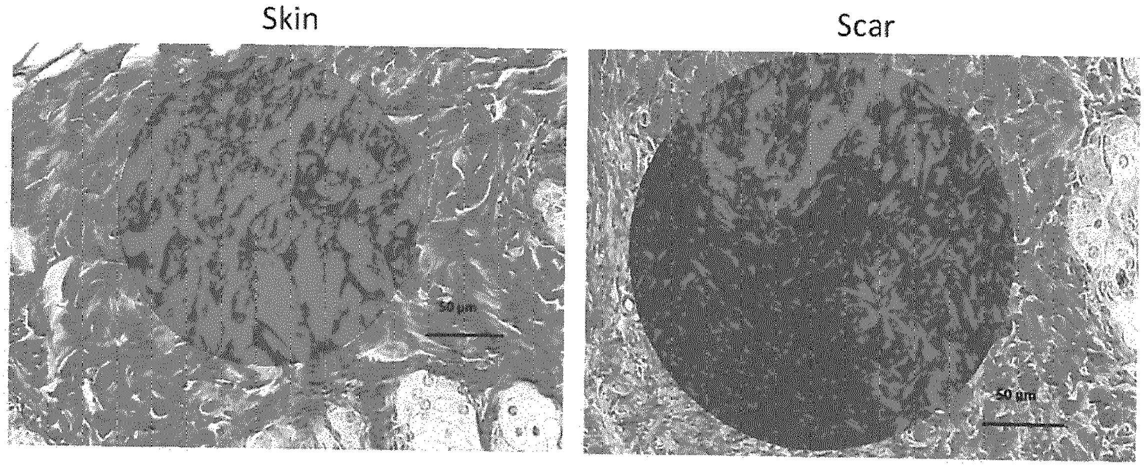


FIG. 7

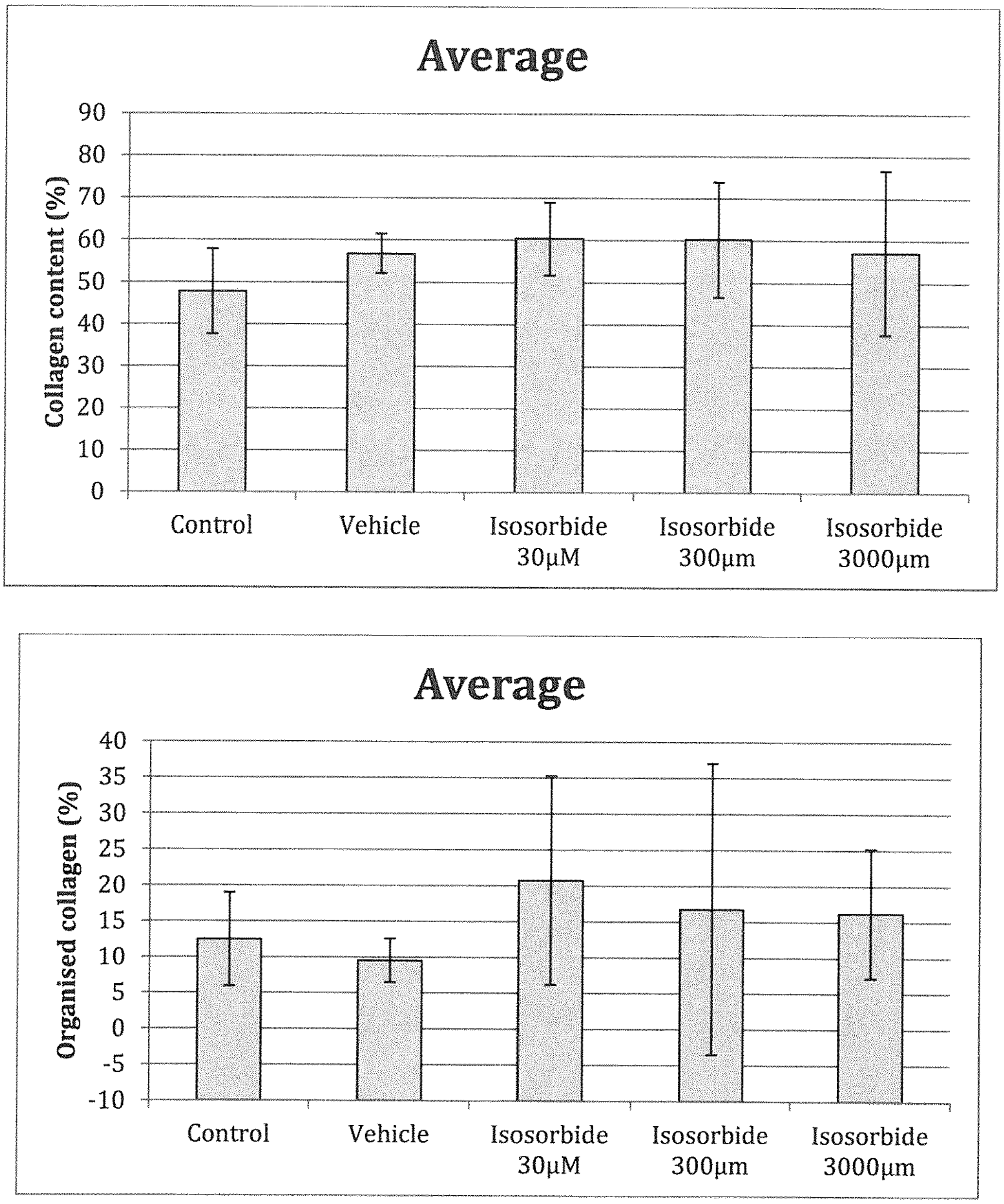
Average
Collagen content (%)
90
80
70
60
50
40
30
20
10
0
Control
Vehicle
Isosorbide 30µM
Isosorbide 300µm
Isosorbide 3000µm
Average
Organised collagen (%)
40
35
30
25
20
15
10
5
0
-5
-10
Control
Vehicle
Isosorbide 30µM
Isosorbide 300µm
Isosorbide 3000µm

FIG. 8

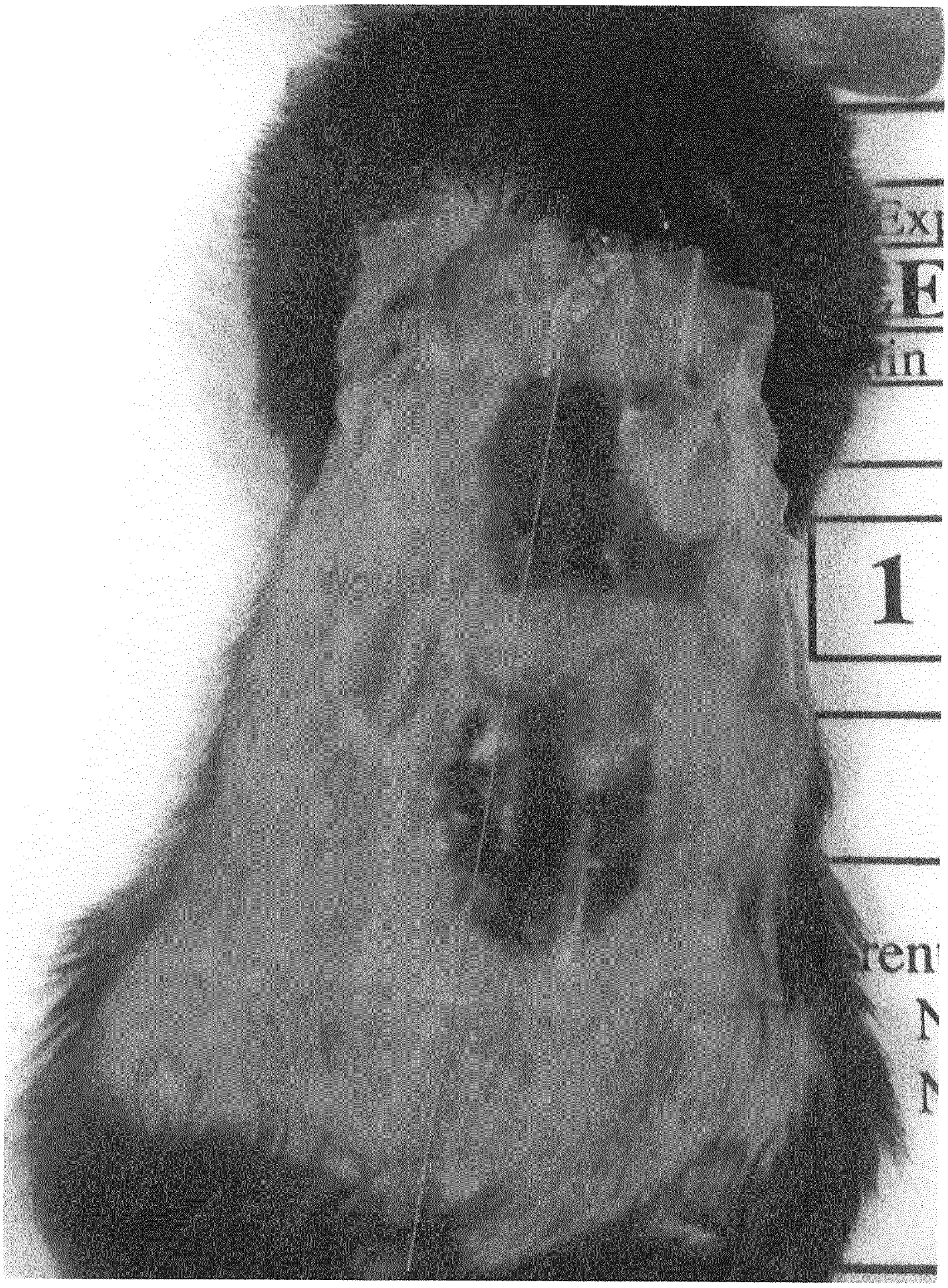

FIG. 9

Europäisches Patentamt
European Patent Office
Office européen des brevets

# EUROPEAN SEARCH REPORT

Application Number
EP 13 18 1571

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/30444 A2 (UNIV JOHNS HOPKINS [US]; SILVER DAVID M [US]; CSUTAK ADRIENNE [HU]; BE) 18 April 2002 (2002-04-18)<br>* paragraphs [0002], [0065] *<br>----- | 1-6,9,10 | INV.<br>A61K9/00<br>A61K9/06<br>A61K31/34<br>A61P17/02 |
| X | WO 2012/131347 A1 (EVOCUTIS PLC [GB]; ABBOTT STEVEN JOHN [GB]; DONOGHUE GAVIN [GB]; EADY) 4 October 2012 (2012-10-04)<br>* page 19, lines 15-24; claim 1 *<br>----- | 1-15 | |

TECHNICAL FIELDS SEARCHED (IPC)
A61K
A61L

The present search report has been drawn up for all claims

1

| Place of search | Date of completion of the search | Examiner |
|---|---|---|
| The Hague | 12 December 2013 | Siebum, Bastiaan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 18 1571

This annex lists the patent family members relating to the patent documents cited in the above-mentioned European search report. The members are as contained in the European Patent Office EDP file on
The European Patent Office is in no way liable for these particulars which are merely given for the purpose of information.

12-12-2013

| Patent document cited in search report | Publication date | Patent family member(s) | Publication date |
|---|---|---|---|
| WO 0230444 A2 | 18-04-2002 | AT 434442 T | 15-07-2009 |
| | | AU 1533402 A | 22-04-2002 |
| | | AU 2002215334 B2 | 10-11-2005 |
| | | CA 2423595 A1 | 18-04-2002 |
| | | DK 1324767 T3 | 10-08-2009 |
| | | EP 1324767 A2 | 09-07-2003 |
| | | ES 2325681 T3 | 14-09-2009 |
| | | JP 2004510825 A | 08-04-2004 |
| | | US 2004001821 A1 | 01-01-2004 |
| | | WO 0230444 A2 | 18-04-2002 |
| WO 2012131347 A1 | 04-10-2012 | WO 2012131347 A1 | 04-10-2012 |
| | | WO 2012131348 A1 | 04-10-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2004127428 A1
- WO 2009043111 A
- US 20040001821 A
- US 2322821 A
- US 2011196161 A
- US 20090301348 A
- WO 2012131348 A
- WO 2005049025 A
- US 2007179121 A
- US 20100322875 A
- WO 2011059866 A
- JP 59175408 A


### Non-patent literature cited in the description


- Remington: The Science and Practice of Pharmacy
- Ansel's Pharmaceutical Dosage Forms and Drug Delivery Systems
- CTFA International Cosmetic Ingredient Dictionary and Handbook. 2004
- **ARCHER, H G ; S BARNETT ; S IRVING ; K R MIDDLETON ; D V SEAL.** A controlled model of moist wound healing: comparison between semi-permeable film, antiseptics and sugar paste. *Journal of experimental pathology (Oxford, England,* 1990, vol. 71 (2), 155-70, http://www.pubmedcentral.nih.gov/articlerender.fcgi?artid=1998714&tool=pmc entrez&rendertype=abstract
- **BÄTZ, FRANZISCA MARIE et al.** Esterase activity in excised and reconstructed human skin - Biotransformation of prednicarbate and the model dye fluorescein diacetate. *European journal of pharmaceutics and biopharmaceutics : official journal of Arbeitsgemeinschaft fur Pharmazeutische Verfahrenstechnik e. V.,* 2012, http://www.ncbi.nlm.nih.gov/pubmed/23201050
- **CHIRIFE, J ; G SCARMATO ; L HERSZAGE.** Scientific basis for use of granulated sugar in treatment of infected wounds. *Lancet,* 1982, vol. 1 (8271), 560-1, http://www.ncbi.nlm.nih.gov/pubmed/6120410
- **CHIRIFE ; JORGE ; L HERSZAGE ; A JOSEPH ; E S KOHN.** In vitro study of bacterial growth inhibition in concentrated sugar solutions: microbiological basis for the use of sugar in treating infected wounds. *Antimicrobial agents and chemotherapy,* 1983, vol. 23 (5), 766-73, http://aac.asm.org/content/23/5/766.short
- **FORREST, R D.** Development of wound therapy from the Dark Ages to the present. *Journal of the Royal Society of Medicine,* 1982, vol. 75 (4), 268-73, http://www.pubmedcentral.nih.gov/articlerender.fcgi?artid=1437659&tool=pmc entrez&rendertype=abstract
- **FORREST, R D.** Early history of wound treatment. *Journal of the Royal Society of Medicine,* 1982, vol. 75 (3), 198-205, http://www.pubmedcentral.nih.gov/articlerender.fcgi?artid=1437561 &tool=pmc entrez&rendertype=abstract
- **KAUFMAN, T ; E H EICHENLAUB ; M F ANGEL ; M LEVIN ; J W FUTRELL.** Topical acidification promotes healing of experimental deep partial thickness skin burns: a randomized double-blind preliminary study. *Burns, including thermal injury,* 1985, vol. 12 (2), 84-90, http://www.sciencedirect.com/science/article/pii/0305417985900324
- **KHORASANI, HOOMAN et al.** A quantitative approach to scar analysis. *The American journal of pathology,* 2011, vol. 178 (2), 621-8, http://www.pubmedcentral.nih.gov/articlerender.fcgi?artid=3070584&tool=pmc entrez&rendertype=abstract
- **KNUTSON, R A ; L A MERBITZ ; M A CREEKMORE ; H G SNIPES.** Use of sugar and povidone-iodine to enhance wound healing: five year's experience. *Southern medical journal,* 1981, vol. 74 (11), 1329-35, http://www.ncbi.nlm.nih.gov/pubmed/7302631
- **KÖSSI, J A ; T O EKFORS ; V AALTONEN ; M LAATO.** Sucrose has no beneficial effects on wound healing in rats. *The European journal of surgery = Acta chirurgica,* 2000, vol. 166 (10), 818-22, http://www.ncbi.nlm.nih.gov/pubmed/11071171

- Chemical acidification of wounds. An adjuvant to healing and the unfavorable action of alkalinity and ammonia. **LEVEEN, H H et al.** Annals of surgery. 1973, vol. 178, 745-53
- **MIYAGAWA, MAIKO et al.** Endolymphatic hydrops and therapeutic effects are visualized in 'atypical' Meniere's disease. *Acta oto-laryngologica,* 2009, vol. 129 (11), 1326-9, http://www.ncbi.nlm.nih.gov/pubmed/19863332
- **NOZAWA, I et al.** Efficacy of long-term administration of isosorbide for Ménière's disease. *ORL; journal for oto-rhino-laryngology and its related specialties,* 1995, vol. 57 (3), 135-40, http://www.ncbi.nlm.nih.gov/pubmed/7603692
- **SINGER, A J ; R A CLARK.** Cutaneous wound healing. *The New England journal of medicine,* 1999, vol. 341 (10), 738-46, http://www.ncbi.nlm.nih.gov/pubmed/10471461
- **TROUILLET, J L et al.** Use of granulated sugar in treatment of open mediastinitis after cardiac surgery. *Lancet,* 1985, vol. 2 (8448), 180-4, http://www.ncbi.nlm.nih.gov/pubmed/2862372
- **DE VISSCHER, GEOFREY et al.** In vivo cellularization of a cross-linked matrix by intraperitoneal implantation: a new tool in heart valve tissue engineering. *European heart journal,* 2007, vol. 28 (11), 1389-96, http://www.ncbi.nlm.nih.gov/pubmed/17244642
- **DE VISSCHER, GEOFREY et al.** Functional and biomechanical evaluation of a completely recellularized stentless pulmonary bioprosthesis in sheep. *The Journal of thoracic and cardiovascular surgery,* 2008, vol. 135 (2), 395-404, http://www.ncbi.nlm.nih.gov/pubmed/18242275